# EUROPEAN PATENT APPLICATION

(11) **EP 4 480 521 A1**
(43) Date of publication of application: **25.12.2024**
(21) Application number: 24183570.1
(22) Date of filing: 20.06.2024
(51) Int. Cl.: A61M 16/00

(54) **VENTILATION DEVICE**

(30) Priority: 21.06.2023 CN 202310748003
(71) Applicant: Shenzhen Mindray Bio-Medical Electronics Co., Ltd., Shenzhen, Guangdong 518057 (CN)
(72) Inventor: LIU, Jinglei, Shenzhen, 518057 (CN); NAN, Zhibai, Shenzhen, 518057 (CN); HUANG, Zhiwen, Shenzhen, 518057 (CN)
(74) Representative: KIPA AB

(57) **Abstract**

This disclosure provides a ventilation device (100) and a method for regulating a gas flow rate thereof. The ventilation device includes:
- a gas source interface (110);
- a patient interface (131);
- a flow rate delivery apparatus (120) which delivers gas to the patient interface;
- a pressure sensor which obtains pressure data of a first signal acquisition point (132a);
- a flow sensor which obtains flow rate data of a second signal acquisition point (132b);
- a processor (170) which controls the flow rate delivery apparatus to deliver gas(es) to the patient interface at multiple first gas flow rates, and calculates a characteristic parameter of flow rate for patient, according to the pressure data and the flow rate data under the multiple first gas flow rates; and controls the flow rate delivery apparatus to deliver gas(es) to the patient interface at a second gas flow rate.
The above ventilation device has a better ventilation effect when providing respiratory support.

## Description

### CROSS-REFERENCE TO RELATED APPLICATION

This disclosure claims priority to Chinese patent Application No. 202310748003.2, filed on June 21, 2023, the content of which is incorporated herein by reference in its entirety.

### TECHNICAL FIELD

The disclosure relates to a technical field of a medical device, and more particularly to a ventilation device, and a method for regulating a gas flow rate of a ventilation device

### BACKGROUND

When providing respiratory support to a patient, a ventilation device often fails to achieve an expected effect due to a gas leakage at a connection point between a patient interface and the patient. Taking high-flow nasal cannula (HFNC) oxygen therapy as an example, through the high-flow nasal cannula oxygen therapy, the patient inhales a high-flow gas with constant temperature and humidity through a nasal catheter. The high-flow gas generates a certain level of positive end expiratory pressure, and washes out a physiological dead space of an upper respiratory tract, which reduces respiratory work of the patient. The inhaled gas can be mixed with a certain concentration of oxygen, thereby improving oxygenation of the patient.

During high-flow oxygen therapy, due to a gas leakage at a connection point between a high-flow nasal cannula and a nasal cavity, oxygen inhaled by the patient is diluted by the air, resulting in that an actual oxygen concentration of the inhaled gas is lower than a preset value of a doctor. Therefore, it is usually necessary to set a gas flow rate for the high-flow oxygen therapy to a value which is higher than an inspiratory peak flow rate of the patient, in order to provide a stable oxygen concentration for the patient.

At present, it is difficult to determine the peak inspiratory flow rate of patient during the high-flow oxygen therapy, which leads a setting of the flow rate for the high-flow oxygen therapy to a challenge in the industry. In the past, doctors mainly relied on experience to estimate the peak inspiratory flow rate of patient and set the flow rate as the estimated value. Some researchers also use a flow meter to measure the peak inspiratory flow rate of patient before undergoing the high-flow oxygen therapy, so as to determine the flow rate. However, this method has low accuracy and is not effective in clinical practice.

### SUMMARY

According to a first aspect, an embodiment provides a ventilation device including:
a gas source interface, which is capable of being connected with a gas source;
a patient interface, which is capable of being connected with a respiratory system of a patient;
a flow rate delivery apparatus, which is configured to deliver gas(es) to the patient interface;
a pressure sensor, which is configured to acquire pressure data of a first signal acquisition point;
a flow sensor, which is configured to acquire flow rate data of a second signal acquisition point; wherein both of the first signal acquisition point and the second acquisition point are positioned between the gas source interface and the patient interface; and
a processor, which is configured to control the flow rate delivery apparatus to deliver gas(es) to the patient interface at multiple first gas flow rates, and calculate a characteristic parameter of flow rate for the patient, according to the pressure data and the flow rate data under the multiple first gas flow rates;
the processor is further configured to control the flow rate delivery apparatus to deliver gas(es) to the patient interface at a second gas flow rate, so as to provide respiratory support to the patient; wherein the second gas flow rate approaches or exceeds a gas flow rate which is inhaled by the patient, when calculating the characteristic parameter of flow rate for the patient;
wherein, the characteristic parameter of flow rate is related to a physiological state of the patient, and used as a reference for determining the second gas flow rate.

In a preferable embodiment, in order to calculate a characteristic parameter of flow rate for the patient, according to the pressure data and the flow rate data under the multiple first gas flow rates, the processor is further configured to:
calculate proximal gas characteristics of a proximal end of the patient under the multiple first gas flow rates, according to the pressure data and the flow rate data under the multiple first gas flow rates; and determine the characteristic parameter of flow rate according to the proximal gas characteristics;
wherein the proximal end of the patient is closer to the patient than the first signal acquisition point and the second signal acquisition point.

In a preferable embodiment, in order to calculate proximal gas characteristics of a proximal end of the patient under the multiple first gas flow rates, according to the pressure data and the flow rate data under the multiple first gas flow rates; and determine the characteristic parameter of flow rate according to the proximal gas characteristics; the processor is further configured to:
calculate a proximal gas characteristic of the proximal end of the patient in a period of time under each first gas flow rate, according to the pressure data and the flow rate data in the period of time under each first gas flow rate; and
determine the characteristic parameter of flow rate according to the proximal gas characteristics in the period of time under the multiple first gas flow rates.

In a preferable embodiment, in order to calculate a proximal gas characteristic of the proximal end of the patient in a period of time under each first gas flow rate, the processor is further configured to:
input the pressure data and the flow rate data in the period of time under each first gas flow rate into a preset model;
calculate a proximal pressure of the proximal end of the patient in the period of time under each first gas flow rate; and
use the proximal pressure as the proximal gas characteristic.

In a preferable embodiment, in order to determine the characteristic parameter of flow rate according to the proximal gas characteristics in the period of time under the multiple first gas flow rates, the processor is further configured to:
calculate a gas characteristic value which corresponds to each first gas flow rate according to the proximal gas characteristic in the period of time under each first gas flow rate;
determine whether the gas characteristic value satisfies a preset condition, and use a first gas flow rate, which corresponds to the gas characteristic value that satisfies the preset condition, as the characteristic parameter of flow rate; or calculate the characteristic parameter of flow rate, according to a first gas flow rate which corresponds to multiple gas characteristic values that satisfy preset condition(s).

In a preferable embodiment, in order to calculate a gas characteristic value which corresponds to each first gas flow rate according to the proximal gas characteristic in the period of time under each first gas flow rate, the processor is further configured to:
perform a statistical analysis on the proximal pressure in the period of time under each first gas flow rate, so as to obtain a pressure statistical value; and use the pressure statistical value as the gas characteristic value; wherein the pressure statistical value is any one of a mean value, a standard deviation value, a median value, a maximum value, and a minimum value.

In a preferable embodiment, in order to determine whether the gas characteristic value satisfies a preset condition, the processor is further configured to:
determine whether the pressure statistical value falls into a preset first threshold range.

In a preferable embodiment, in order to calculate a gas characteristic value which corresponds to each first gas flow rate according to the proximal gas characteristic in the period of time under each first gas flow rate, the processor is further configured to:
record a first time length for the flow rate delivery apparatus to deliver gas(es) to the patient interface at each first gas flow rate;
record a second time length in which the proximal pressure exceeds a preset pressure threshold, when the flow rate delivery apparatus delivers gas(es) to the patient interface at each first gas flow rate; and
use a ratio of the second time length to the first time length as the gas characteristic value.

In a preferable embodiment, in order to determine whether the gas characteristic value satisfies a preset condition, the processor is further configured to:
determine whether the ratio falls into a preset second threshold range.

In a preferable embodiment, in order to calculate a gas characteristic value which corresponds to each first gas flow rate according to the proximal gas characteristic in the period of time under each first gas flow rate, the processor is further configured to:
obtain an integral value of the proximal pressure over time during an inspiratory phase of the patient, when the flow rate delivery apparatus delivers gas(es) to the patient interface at each first gas flow rate; and
use the integral value as the gas characteristic value.

In a preferable embodiment, in order to determine whether the gas characteristic value satisfies a preset condition, the processor is further configured to:
determine whether the integral value falls into a preset third threshold range.

In a preferable embodiment, the processor is further configured to, at a predetermined interval, repeatedly:
control the flow rate delivery apparatus to deliver gas(es) to the patient interface at the multiple first gas flow rates, and calculate the characteristic parameter of flow rate according to the pressure data and the flow rate data under the multiple first gas flow rates.

In a preferable embodiment, in order to control the flow rate delivery apparatus to deliver gas(es) to the patient interface at the multiple first gas flow rates, and calculate the characteristic parameter of flow rate according to the pressure data and the flow rate data under the multiple first gas flow rates, the processor is further configured to:
compare the flow rate data with a fourth threshold range, when the flow rate delivery apparatus delivers gas(es) to the patient interface at the second gas flow rate to provide respiratory support to the patient; and
control the flow rate delivery apparatus to deliver gas(es) to the patient interface at the multiple first gas flow rates, and calculate the characteristic parameter of flow rate according to the pressure data and the flow rate data under the multiple first gas flow rates, when the flow rate data is determined to exceed the fourth threshold range.

In a preferable embodiment, in order to control the flow rate delivery apparatus to deliver gas(es) to the patient interface at the multiple first gas flow rates, and calculate the characteristic parameter of flow rate according to the pressure data and the flow rate data under the multiple first gas flow rates, the processor is further configured to:
compare the pressure data with a fifth threshold range, when the flow rate delivery apparatus delivers gas(es) to the patient interface at the second gas flow rate to provide respiratory support to the patient; and
control the flow rate delivery apparatus to deliver gas(es) to the patient interface at the multiple first gas flow rates, and calculate the characteristic parameter of flow rate according to the pressure data and the flow rate data under the multiple first gas flow rates, when the pressure data is determined to exceed the fifth threshold range.

In a preferable embodiment, the processor is further configured to:
set the second gas flow rate according to the characteristic parameter of flow rate.

In a preferable embodiment, in order to set the second gas flow rate according to the characteristic parameter of flow rate, the processor is further configured to:
obtain a change value of flow rate, and take a sum of the characteristic parameter of flow rate and the change value of flow rate as the second gas flow rate.

In a preferable embodiment, in order to set the second gas flow rate according to the characteristic parameter of flow rate, the processor is further configured to:
obtain a proportional parameter, and take a product of the characteristic parameter of flow rate and the proportional parameter as the second gas flow rate.

In a preferable embodiment, in order to set the second gas flow rate according to the characteristic parameter of flow rate, the processor is further configured to:
set the second gas flow rate according to a changed characteristic parameter of flow rate, after determining that the characteristic parameter of flow rate has been changed.

In a preferable embodiment, using the characteristic parameter of flow rate as a reference for determining the second gas flow rate, includes at least one of:
displaying the characteristic parameter of flow rate;
outputting information, which is related to the characteristic parameter of flow rate, so as to indicate to a user to set the second gas flow rate; and
outputting information, which is related to the changed characteristic parameter of flow rate, after the characteristic parameter of flow rate is determined to have been changed, so as to indicate to a user to increase or decrease the second gas flow rate.

In a preferable embodiment, in a first ventilation phase, the processor is further configured to control the ventilation device to deliver gas(es) to the patient interface at the multiple first gas flow rates, and to calculate the characteristic parameter of flow rate for the patient according to the pressure data and the flow rate data under the multiple first gas flow rates;
in a second ventilation phase, the processor is further configured to control the flow rate delivery apparatus to deliver gas(es) to the patient interface at the second gas flow rate, so as to provide respiratory support to the patient.

In a preferable embodiment, the ventilation device further includes a housing; wherein the processor, the pressure sensor, and the flow sensor are all positioned within the housing.

According to a second aspect, an embodiment provides a method for regulating a gas flow rate of a ventilation device, including:
acquiring pressure data of a first signal acquisition point;
acquiring flow rate data of a second signal acquisition point; wherein both of the first signal acquisition point and the second acquisition point are positioned between a gas source interface of the ventilation device and a patient interface of the ventilation device; wherein the gas source interface is capable of being connected with a gas source; the patient interface is capable of being connected with a respiratory system of a patient;
delivering gas(es) to the patient interface at multiple first gas flow rates; and calculating a characteristic parameter of flow rate for the patient, according to the pressure data and the flow rate data under the multiple first gas flow rates;
delivering gas(es) to the patient interface at a second gas flow rate, so as to provide respiratory support to the patient, wherein the second gas flow rate approaches or exceeds a gas flow rate which is inhaled by the patient, when calculating the characteristic parameter of flow rate for the patient; and
wherein, the characteristic parameter of flow rate is related to a physiological state of the patient, and used as a reference for determining the second gas flow rate.

In a preferable embodiment, calculating a characteristic parameter of flow rate for the patient, according to the pressure data and the flow rate data under the multiple first gas flow rates, includes:
calculating proximal gas characteristics of a proximal end of the patient under the multiple first gas flow rates, according to the pressure data and the flow rate data under the multiple first gas flow rates; and determining the characteristic parameter of flow rate according to the proximal gas characteristics;
wherein the proximal end of the patient is closer to the patient than the first signal acquisition point and the second signal acquisition point.

In a preferable embodiment, calculating proximal gas characteristics of a proximal end of the patient under the multiple first gas flow rates, according to the pressure data and the flow rate data under the multiple first gas flow rates; and determining the characteristic parameter of flow rate according to the proximal gas characteristics, includes:
calculating a proximal gas characteristic of the proximal end of the patient in a period of time under each first gas flow rate, according to the pressure data and the flow rate data in the period of time under each first gas flow rate; and
determining the characteristic parameter of flow rate according to the proximal gas characteristics in the period of time under the multiple first gas flow rates.

In a preferable embodiment, calculating a proximal gas characteristic of the proximal end of the patient in a period of time under each first gas flow rate, includes:
inputting the pressure data and the flow rate data in the period of time under each first gas flow rate into a preset model;
calculating a proximal pressure of the proximal end of the patient in the period of time under each first gas flow rate; and
using the proximal pressure as the proximal gas characteristic.

In a preferable embodiment, determining the characteristic parameter of flow rate according to the proximal gas characteristics in the period of time under the multiple first gas flow rates, includes:
calculating a gas characteristic value which corresponds to each first gas flow rate according to the proximal gas characteristic in the period of time under each first gas flow rate;
determining whether the gas characteristic value satisfies a preset condition, and using a first gas flow rate, which corresponds to the gas characteristic value that satisfies the preset condition, as the characteristic parameter of flow rate; or calculating the characteristic parameter of flow rate, according to a first gas flow rate which corresponds to multiple gas characteristic values that satisfy preset condition(s).

In a preferable embodiment, the method further includes repeating following steps at a predetermined interval:
delivering gas(es) to the patient interface at the multiple first gas flow rates, and calculating the characteristic parameter of flow rate according to the pressure data and the flow rate data under the multiple first gas flow rates.

In a preferable embodiment, delivering gas(es) to the patient interface at the multiple first gas flow rates, and calculating the characteristic parameter of flow rate according to the pressure data and the flow rate data under the multiple first gas flow rates, includes:
comparing the flow rate data with a fourth threshold range, when gas(es) is(are) delivered to the patient interface at the second gas flow rate to provide respiratory support to the patient; and
delivering gas(es) to the patient interface at the multiple first gas flow rates, and calculating the characteristic parameter of flow rate according to the pressure data and the flow rate data under the multiple first gas flow rates, when the flow rate data is determined to exceed the fourth threshold range.

In a preferable embodiment, delivering gas(es) to the patient interface at the multiple first gas flow rates, and calculating the characteristic parameter of flow rate according to the pressure data and the flow rate data under the multiple first gas flow rates, includes:
comparing the pressure data with a fifth threshold range, when gas(es) is(are) delivered to the patient interface at the second gas flow rate to provide respiratory support to the patient; and
delivering gas(es) to the patient interface at the multiple first gas flow rates, and calculating the characteristic parameter of flow rate according to the pressure data and the flow rate data under the multiple first gas flow rates, when the pressure data is determined to exceed the fifth threshold range.

In a preferable embodiment, the method further includes setting the second gas flow rate according to the characteristic parameter of flow rate.

In a preferable embodiment, using the characteristic parameter of flow rate as a reference for determining the second gas flow rate, includes at least one of:
displaying the characteristic parameter of flow rate;
outputting information, which is related to the characteristic parameter of flow rate, so as to indicate to a user to set the second gas flow rate; and
outputting information, which is related to the changed characteristic parameter of flow rate, after the characteristic parameter of flow rate is determined to have been changed, so as to indicate to a user to increase or decrease the second gas flow rate.

In a preferable embodiment, the method further includes:
in a first ventilation phase, delivering gas(es) to the patient interface at the multiple first gas flow rates, and calculating the characteristic parameter of flow rate for the patient according to the pressure data and flow rate data under the multiple first gas flow rates;
in a second ventilation phase, delivering gas(es) to the patient interface at the second gas flow rate, so as to provide respiratory support to the patient.

According to a third aspect, an embodiment provides a ventilation device including:
a gas source interface, which is capable of being connected with a gas source;
a patient interface, which is capable of being connected with a respiratory system of a patient;
a flow rate delivery apparatus, which is configured to deliver gas(es) to the patient interface; and
a processor, which is configured to:
   acquire pressure data of a first signal acquisition point, and acquire flow rate data of a second signal acquisition point; wherein both of the first signal acquisition point and the second acquisition point are positioned between the gas source interface and the patient interface; and
   control the flow rate delivery apparatus to deliver gas(es) to the patient interface at multiple first gas flow rates, and calculate a characteristic parameter of flow rate of a patient, according to the pressure data and the flow rate data under the multiple first gas flow rates; wherein the characteristic parameter of flow rate is related to a physiological state of the patient.

In a preferable embodiment, in order to calculate a characteristic parameter of flow rate for the patient, according to the pressure data and the flow rate data under the multiple first gas flow rates, the processor is further configured to:
calculate proximal gas characteristics of a proximal end of the patient under the multiple first gas flow rates, according to the pressure data and the flow rate data under the multiple first gas flow rates; and determine the characteristic parameter of flow rate according to the proximal gas characteristics;
wherein the proximal end of the patient is closer to the patient than the first signal acquisition point and the second signal acquisition point.

In a preferable embodiment, in order to calculate proximal gas characteristics of a proximal end of the patient under the multiple first gas flow rates, according to the pressure data and the flow rate data under the multiple first gas flow rates; and determine the characteristic parameter of flow rate according to the proximal gas characteristics; the processor is further configured to:
calculate a proximal gas characteristic of the proximal end of the patient in a period of time under each first gas flow rate, according to the pressure data and the flow rate data in the period of time under each first gas flow rate; and
determine the characteristic parameter of flow rate according to the proximal gas characteristics in the period of time under the multiple first gas flow rates.

In a preferable embodiment, in order to calculate a proximal gas characteristic of the proximal end of the patient in a period of time under each first gas flow rate, the processor is further configured to:
input the pressure data and the flow rate data in the period of time under each first gas flow rate into a preset model;
calculate a proximal pressure of the proximal end of the patient in the period of time under each first gas flow rate; and
use the proximal pressure as the proximal gas characteristic.

In a preferable embodiment, in order to determine the characteristic parameter of flow rate according to the proximal gas characteristics in the period of time under the multiple first gas flow rates, the processor is further configured to:
calculate a gas characteristic value which corresponds to each first gas flow rate according to the proximal gas characteristic in the period of time under each first gas flow rate;
determine whether the gas characteristic value satisfies a preset condition, and use a first gas flow rate, which corresponds to the gas characteristic value that satisfies the preset condition, as the characteristic parameter of flow rate; or calculate the characteristic parameter of flow rate, according to a first gas flow rate which corresponds to multiple gas characteristic values that satisfy preset condition(s).

In a preferable embodiment, in order to calculate a gas characteristic value which corresponds to each first gas flow rate according to the proximal gas characteristic in the period of time under each first gas flow rate, the processor is further configured to:
perform a statistical analysis on the proximal pressure in the period of time under each first gas flow rate, so as to obtain a pressure statistical value; and use the pressure statistical value as the gas characteristic value; wherein the pressure statistical value is any one of a mean value, a standard deviation value, a median value, a maximum value, and a minimum value.

In a preferable embodiment, in order to determine whether the gas characteristic value satisfies a preset condition, the processor is further configured to:
determine whether the pressure statistical value falls into a preset first threshold range.

In a preferable embodiment, in order to calculate a gas characteristic value which corresponds to each first gas flow rate according to the proximal gas characteristic in the period of time under each first gas flow rate, the processor is further configured to:
record a first time length for the flow rate delivery apparatus to deliver gas(es) to the patient interface at each first gas flow rate;
record a second time length in which the proximal pressure exceeds a preset pressure threshold, when the flow rate delivery apparatus delivers gas(es) to the patient interface at each first gas flow rate; and
use a ratio of the second time length to the first time length as the gas characteristic value.

In a preferable embodiment, in order to determine whether the gas characteristic value satisfies a preset condition, the processor is further configured to:
determine whether the ratio falls into a preset second threshold range.

In a preferable embodiment, in order to calculate a gas characteristic value which corresponds to each first gas flow rate according to the proximal gas characteristic in the period of time under each first gas flow rate, the processor is further configured to:
obtain an integral value of the proximal pressure over time during an inspiratory phase of the patient, when the flow rate delivery apparatus delivers gas(es) to the patient interface at each first gas flow rate; and
use the integral value as the gas characteristic value.

In a preferable embodiment, in order to determine whether the gas characteristic value satisfies a preset condition, the processor is further configured to:
determine whether the integral value falls into a preset third threshold range.

In a preferable embodiment, the processor is further configured to, at a predetermined interval, repeatedly:
control the flow rate delivery apparatus to deliver gas(es) to the patient interface at the multiple first gas flow rates, and calculate the characteristic parameter of flow rate according to the pressure data and the flow rate data under the multiple first gas flow rates.

In a preferable embodiment, the processor is further configured to control the flow rate delivery apparatus to deliver gas(es) to the patient interface at a second gas flow rate, so as to provide respiratory support to the patient; wherein the second gas flow rate approaches or exceeds a gas flow rate which is inhaled by the patient, when calculating the characteristic parameter of flow rate for the patient; wherein, the characteristic parameter of flow rate used as a reference for determining the second gas flow rate.

In a preferable embodiment, in order to control the flow rate delivery apparatus to deliver gas(es) to the patient interface at the multiple first gas flow rates, and calculate the characteristic parameter of flow rate according to the pressure data and the flow rate data under the multiple first gas flow rates, the processor is further configured to:
compare the flow rate data with a fourth threshold range, when the flow rate delivery apparatus delivers gas(es) to the patient interface at the second gas flow rate to provide respiratory support to the patient; and
control the flow rate delivery apparatus to deliver gas(es) to the patient interface at the multiple first gas flow rates, and calculate the characteristic parameter of flow rate according to the pressure data and the flow rate data under the multiple first gas flow rates, when the flow rate data is determined to exceed the fourth threshold range.

In a preferable embodiment, in order to control the flow rate delivery apparatus to deliver gas(es) to the patient interface at the multiple first gas flow rates, and calculate the characteristic parameter of flow rate according to the pressure data and the flow rate data under the multiple first gas flow rates, the processor is further configured to:
compare the pressure data with a fifth threshold range, when the flow rate delivery apparatus delivers gas(es) to the patient interface at the second gas flow rate to provide respiratory support to the patient; and
control the flow rate delivery apparatus to deliver gas(es) to the patient interface at the multiple first gas flow rates, and calculate the characteristic parameter of flow rate according to the pressure data and the flow rate data under the multiple first gas flow rates, when the pressure data is determined to exceed the fifth threshold range.

In a preferable embodiment, the processor is further configured to set the second gas flow rate according to the characteristic parameter of flow rate.

In a preferable embodiment, in order to set the second gas flow rate according to the characteristic parameter of flow rate, the processor is further configured to:
obtain a change value of flow rate, and take a sum of the characteristic parameter of flow rate and the change value of flow rate as the second gas flow rate.

In a preferable embodiment, in order to set the second gas flow rate according to the characteristic parameter of flow rate, the processor is further configured to:
obtain a proportional parameter, and take a product of the characteristic parameter of flow rate and the proportional parameter as the second gas flow rate.

In a preferable embodiment, in order to set the second gas flow rate according to the characteristic parameter of flow rate, the processor is further configured to:
set the second gas flow rate according to a changed characteristic parameter of flow rate, after determining that the characteristic parameter of flow rate has been changed.

In a preferable embodiment, using the characteristic parameter of flow rate as a reference for determining the second gas flow rate, includes at least one of:
displaying the characteristic parameter of flow rate;
outputting information, which is related to the characteristic parameter of flow rate, so as to indicate to a user to set the second gas flow rate; and
outputting information, which is related to the changed characteristic parameter of flow rate, after the characteristic parameter of flow rate is determined to have been changed, so as to indicate to a user to increase or decrease the second gas flow rate.

In a preferable embodiment, in a first ventilation phase, the processor is further configured to control the ventilation device to deliver gas(es) to the patient interface at the multiple first gas flow rates, and to calculate the characteristic parameter of flow rate for the patient according to the pressure data and the flow rate data under the multiple first gas flow rates;
in a second ventilation phase, the processor is further configured to control the flow rate delivery apparatus to deliver gas(es) to the patient interface at the second gas flow rate, so as to provide respiratory support to the patient.

In a preferable embodiment, the processor is further configured to compare the characteristic parameter of flow rate with a preset parameter threshold, and send an alarm when the characteristic parameter of flow rate does not match with the parameter threshold.

In a preferable embodiment, the ventilation device further includes a pressure sensor and/or a flow sensor; wherein the pressure sensor is configured to acquire the pressure data of the first signal acquisition point, and the flow sensor is configured to acquire the flow rate data of the second signal acquisition point.

In a preferable embodiment, the ventilation device further includes a housing; wherein the processor, and the pressure sensor and/or the flow sensor, are all positioned within the housing.

According to a fourth aspect, an embodiment provides a method for regulating a gas flow rate of a ventilation device, including:
acquiring pressure data of a first signal acquisition point;
acquiring flow rate data of a second signal acquisition point; wherein both of the first signal acquisition point and the second acquisition point are positioned between a gas source interface of the ventilation device and a patient interface of the ventilation device; wherein the gas source interface is capable of being connected with a gas source; the patient interface is capable of being connected with a respiratory system of a patient;
delivering gas(es) to the patient interface at a second gas flow rate, so as to provide respiratory support to the patient, wherein the second gas flow rate approaches or exceeds a gas flow rate which is inhaled by the patient, when calculating a characteristic parameter of flow rate for the patient; and
delivering gas(es) to the patient interface at multiple first gas flow rates; and calculating a characteristic parameter of flow rate for the patient, according to the pressure data and the flow rate data under the multiple first gas flow rates;
wherein, the characteristic parameter of flow rate is related to a physiological state of the patient.

In a preferable embodiment, calculating a characteristic parameter of flow rate for the patient, according to the pressure data and the flow rate data under the multiple first gas flow rates, includes:
calculating proximal gas characteristics of a proximal end of the patient under the multiple first gas flow rates, according to the pressure data and the flow rate data under the multiple first gas flow rates; and determining the characteristic parameter of flow rate according to the proximal gas characteristics;
wherein the proximal end of the patient is closer to the patient than the first signal acquisition point and the second signal acquisition point.

In a preferable embodiment, calculating proximal gas characteristics of a proximal end of the patient under the multiple first gas flow rates, according to the pressure data and the flow rate data under the multiple first gas flow rates; and determining the characteristic parameter of flow rate according to the proximal gas characteristics, includes:
calculating a proximal gas characteristic of the proximal end of the patient in a period of time under each first gas flow rate, according to the pressure data and the flow rate data in the period of time under each first gas flow rate; and
determining the characteristic parameter of flow rate according to the proximal gas characteristics in the period of time under the multiple first gas flow rates.

In a preferable embodiment, calculating a proximal gas characteristic of the proximal end of the patient in a period of time under each first gas flow rate, includes:
inputting the pressure data and the flow rate data in the period of time under each first gas flow rate into a preset model;
calculating a proximal pressure of the proximal end of the patient in the period of time under each first gas flow rate; and
using the proximal pressure as the proximal gas characteristic.

In a preferable embodiment, determining the characteristic parameter of flow rate according to the proximal gas characteristics in the period of time under the multiple first gas flow rates, includes:
calculating a gas characteristic value which corresponds to each first gas flow rate according to the proximal gas characteristic in the period of time under each first gas flow rate;
determining whether the gas characteristic value satisfies a preset condition, and using a first gas flow rate, which corresponds to the gas characteristic value that satisfies the preset condition, as the characteristic parameter of flow rate; or calculating the characteristic parameter of flow rate, according to a first gas flow rate which corresponds to multiple gas characteristic values that satisfy preset condition(s).

In a preferable embodiment, the method further includes repeating following steps at a predetermined interval:
delivering gas(es) to the patient interface at the multiple first gas flow rates, and calculating the characteristic parameter of flow rate according to the pressure data and the flow rate data under the multiple first gas flow rates.

In a preferable embodiment, the method further includes delivering gas(es) to the patient interface at a second gas flow rate, so as to provide respiratory support to the patient; wherein the second gas flow rate approaches or exceeds a gas flow rate which is inhaled by the patient, when calculating the characteristic parameter of flow rate for the patient; wherein, the characteristic parameter of flow rate used as a reference for determining the second gas flow rate.

In a preferable embodiment, delivering gas(es) to the patient interface at the multiple first gas flow rates, and calculating the characteristic parameter of flow rate according to the pressure data and the flow rate data under the multiple first gas flow rates, includes:
comparing the flow rate data with a fourth threshold range, when gas(es) is(are) delivered to the patient interface at the second gas flow rate to provide respiratory support to the patient; and
delivering gas(es) to the patient interface at the multiple first gas flow rates, and calculating the characteristic parameter of flow rate according to the pressure data and the flow rate data under the multiple first gas flow rates, when the flow rate data is determined to exceed the fourth threshold range.

In a preferable embodiment, delivering gas(es) to the patient interface at the multiple first gas flow rates, and calculating the characteristic parameter of flow rate according to the pressure data and the flow rate data under the multiple first gas flow rates, includes:
comparing the pressure data with a fifth threshold range, when gas(es) is(are) delivered to the patient interface at the second gas flow rate to provide respiratory support to the patient; and
delivering gas(es) to the patient interface at the multiple first gas flow rates, and calculating the characteristic parameter of flow rate according to the pressure data and the flow rate data under the multiple first gas flow rates, when the pressure data is determined to exceed the fifth threshold range.

In a preferable embodiment, the method further includes setting the second gas flow rate according to the characteristic parameter of flow rate.

In a preferable embodiment, using the characteristic parameter of flow rate as a reference for determining the second gas flow rate, includes at least one of:
displaying the characteristic parameter of flow rate;
outputting information, which is related to the characteristic parameter of flow rate, so as to indicate to a user to set the second gas flow rate; and
outputting information, which is related to the changed characteristic parameter of flow rate, after the characteristic parameter of flow rate is determined to have been changed, so as to indicate to a user to increase or decrease the second gas flow rate.

In a preferable embodiment, the method further includes in a first ventilation phase, delivering gas(es) to the patient interface at the multiple first gas flow rates, and calculating the characteristic parameter of flow rate for the patient according to the pressure data and flow rate data under the multiple first gas flow rates;
in a second ventilation phase, delivering gas(es) to the patient interface at the second gas flow rate, so as to provide respiratory support to the patient.

In a preferable embodiment, the method further includes comparing the characteristic parameter of flow rate with a preset parameter threshold, and sending an alarm when the characteristic parameter of flow rate does not match with the parameter threshold.

According to a fifth aspect, an embodiment provides a computer-readable storage medium, on which program(s) is(are) stored, wherein the program program(s) is(are) capable of being executed by a processor to implement the above method.

According to the ventilation device and the method for regulating a gas flow rate in the above embodiments, pressure data are obtained at a first signal acquisition point between a gas source interface and a patient interface, and flow rate data are obtained at a second signal acquisition point between the gas source interface and the patient interface. During patient ventilation, multiple first gas flow rates are used to deliver gas(es) to the patient, and a characteristic parameter of flow rate for the patient is calculated according to the pressure data and the flow rate data under the multiple first gas flow rates. This characteristic parameter of flow rate can be used as a reference for determining the second gas flow rate, which is configured to provide respiratory support. The above process can be completed during the ventilation process. As the characteristic parameter of flow rate is calculated during the ventilation process, using the characteristic parameter of flow rate as a reference to determine the second gas flow rate can better satisfy actual requirement of patient. For example, during a period of high-flow oxygen therapy for a patient, gas(es) can be delivered at multiple first gas flow rates, and then the appropriate second gas flow rate for oxygen therapy can be determined based on the obtained characteristic parameter of flow rate, thereby providing stable oxygen concentration for patient, and ensuring ventilation effectiveness.

### BRIEF DESCRIPTION OF THE DRAWINGS

FIG. 1 is a structural diagram of a ventilation device in an embodiment.
FIG. 2 is a diagram of a relationship between a pressure difference and flow rate data in an embodiment.
FIG. 3 is a curve graph of pressure data, flow rate data, and proximal pressure over time in an embodiment.
FIG. 4 is a curve graph of a proximal pressure which changes with time under multiple first gas flow rates in an embodiment.
FIG. 5 is a curve graph of a proximal pressure which changes with time under one first gas flow rate in an embodiment.
FIG. 6 is a curve graph of a proximal pressure which changes with time under multiple first gas flow rates in another embodiment.
FIG. 7 is a flow chart of a method for regulating a gas flow rate of a ventilation device in an embodiment.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted sequentially in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the description are operable to distinguish different objects, rather than to describe a specific order. In addition, the terms "connection", and "linkage" mentioned in this disclosure include direct and indirect connection (linkage), unless otherwise specified.

The term "providing respiratory support to a patient" in this disclosure refers to delivering gas(es) to a patient with a purpose of assisting a respiration of the patient. For example, delivering gas(es) to a patient during a high-flow nasal cannula oxygen therapy is for providing respiratory support.

At present, ventilation mainly includes invasive ventilation and non-invasive ventilation. Wherein, non-invasive ventilation provides ventilation to the patient, through connecting components such as masks and nasal catheters, to a part of a patient, such as a mouth, nose and so on. These components are collectively referred to as a patient interface in some embodiments below. Gas leakage is inevitable at a connection point between the patient interface and the patient, which results in an error between the expected gas flow rate delivered, and the actual gas flow rate that the patient can inhale when providing respiratory support to the patient. For example, medical staff estimate a current gas flow rate that the patient needs to inhale as N, and according to this, set a gas flow rate delivered by the ventilation device as M, wherein M>N. Due to gas leakage at the connection point between the patient interface and the patient, an actual gas flow rate that the patient can inhale, changes from M to m, wherein m<N, then the gas flow rate for the patient is insufficient. Therefore, at least in non-invasive ventilation, gas leakage should be considered to set the gas flow rate for delivery. The gas flow rate of the ventilation device set by medical staff should be greater than a maximum gas flow rate required by the patient. In the following text, take that a gas flow rate set by the ventilation device needs to be greater than a peak inspiratory flow rate of the patient as an example for explanation.

The inventor found in practice that it is not easy to determine the peak inspiratory flow rate for a patient who receives respiratory support. The peak inspiratory flow rate measured by a flow meter or other methods before the patient receives respiratory support may differ significantly from a peak inspiratory flow rate which is obtained when the patient undergoes respiratory support. Therefore, the best practice is to measure the peak inspiratory flow rate of the patient when the patient undergoes the respiratory support. The most important concept of this disclosure is that a gas flow rate required by the patient can be obtained during continuous respiratory support, without interrupting a ventilation of a ventilation device or reconnecting the patient interface with the patient, so as to minimize factor(s) affecting the gas flow rate required by the patient. Then, the ventilation device can be set more accurately according to the gas flow rate required by the patient.

It should also be noted that another significance of accurately measuring the gas flow rate that a patient needs to inhale during respiratory support, is that the gas flow rate of the ventilation device cannot be set too high. For example, when it is found that a gas flow rate currently set exceeds too much than a gas flow rate that a patient needs to inhale, the gas flow rate of the ventilation device can be appropriately reduced to avoid adverse effects on the respiration of the patient caused by excessive gas flow rate.

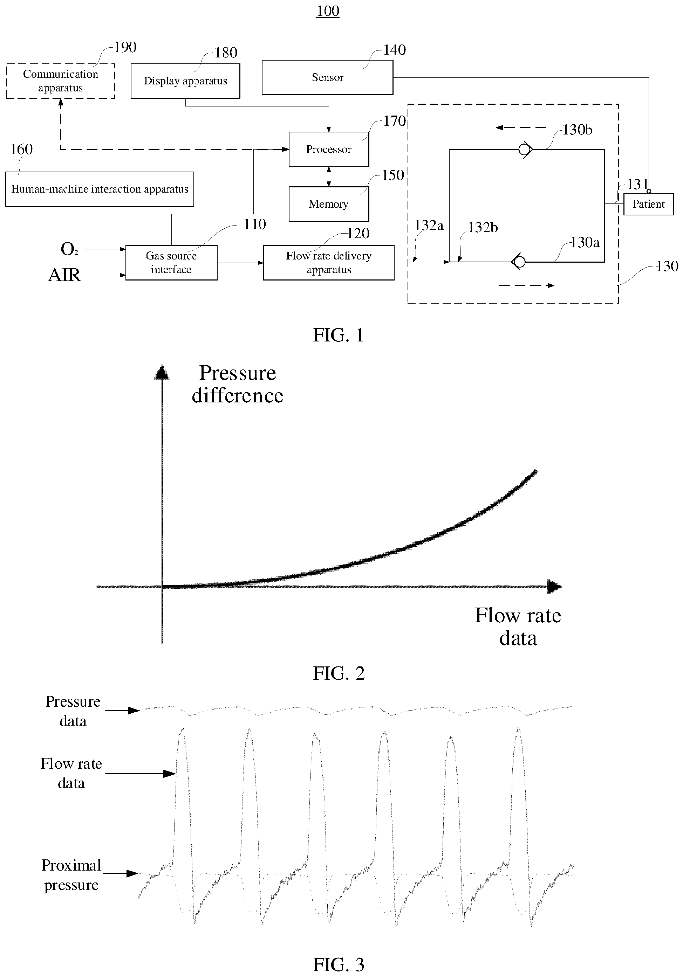

Please refer to a ventilation device 100 shown in FIG. 1. The ventilation device 100 is configured to provide respiratory support for a patient. The ventilation device 100 can include but is not limited to a ventilator, an anesthesia machine, and an oxygen therapy device. Respiratory support includes but is not limited to oxygen therapy. For example, the ventilation device 100 can perform high-flow (nasal cannula) oxygen therapy or other non-invasive ventilation therapy on a patient.

Specifically, the ventilation device 100 may include a gas source interface 110, a flow rate delivery apparatus 120, a respiratory pipeline 130, a sensor 140, a memory 150, a human-machine interaction apparatus 160, a processor 170, and a display apparatus 180. It should be understood that FIG. 1 is only an example of ventilation device 100 and does not constitute a limitation on the ventilation device 100. When the ventilation device 100 is a specific type of instrument, it may include more or fewer components than those shown in FIG. 1, or combine certain components of those shown in FIG. 1, or include components different from those shown in FIG. 1. For example, when the ventilation device 100 is an oxygen therapy device that provides a patient with high-flow nasal cannula oxygen therapy (hereinafter referred to as HFNC), the oxygen therapy device may also include a gas heating apparatus and a gas humidification apparatus. The respiratory pipeline 130 can be a single pipeline or a dual pipeline (as shown in FIG. 1).

The gas source interface 110 is connected with a gas source (not shown), which is configured to provide gas(es) which usually use(s) oxygen and air, etc. In some embodiments, the gas source can be a compressed gas cylinder or a central gas supply, with types of gas supply including oxygen O₂ and air, etc. In other embodiments, the gas source can also be atmospheric gas, such as air in the external environment. That is to say, the ventilation device 100 can be a pneumatic, electric, or hybrid power device. The gas source interface 110 may include conventional components, such as a pressure gauge, a pressure regulator, a flow meter, and a pressure reducing valve, which are respectively configured to control respective flow rates of various gases (such as oxygen and air). A gas, which is inputted from the gas source interface 110, enters the flow rate delivery apparatus 120 and respiratory pipeline 130 and is outputted to the patient interface 131.

The flow rate delivery apparatus 120 is configured to drive the gas which is inputted from the gas source interface 110 to the respiratory system of a patient at a specific flow rate. In specific embodiments, the flow rate delivery apparatus 120 typically includes a computer-controlled ventilation module, and a gas flow pipeline of the computer-controlled ventilation module is connected with the respiratory pipeline 130. In some embodiments, the flow rate delivery apparatus 120 further includes a turbine.

In this disclosure, the respiratory pipeline 130 can be a dual pipeline, which includes an inspiratory pipeline 130a and an expiratory pipeline 130b. A mixed gas of fresh air, which air is introduced by the gas source interface 110, is inputted from an inlet of the inspiratory pipeline 130a and provided to the patient through the patient interface 131, wherein the patient interface 131 is arranged at an outlet of the inspiratory pipeline 130a. In some embodiments, a peak flow velocity of the patient can be estimated by measuring a flow rate of the expiratory pathway 140b. In other embodiments, the respiratory pipeline 130 can also be a single pipeline, for example, the patient interface 131 is a mask worn on the face of a patient, and the single pipeline deliver a respiratory gas into the mask for the patient to inhale, while the exhaled gas is directly discharged through the mask or an expiratory valve. In addition, when the ventilation device 100 is an oxygen therapy device, the patient interface 131 can be a nasal catheter for inserting into the nose of a patient.

A sensor 140 is configured to acquire, in real-time, a physiological parameter of a patient during ventilation therapy and/or a ventilation parameter of the ventilation device 100. One or more sensors 140 exist, and the physiological parameter may correspondingly include a blood oxygen parameter and a respiratory parameter. Wherein the blood oxygen parameter may include but is not limited to percutaneous blood oxygen saturation (SpO2), partial pressure of arterial blood oxygen (Pa02), and arterial blood oxygen saturation (SaO2), while the respiratory parameter may include but is not limited to partial pressure of inspiration oxygen (FiO2) and respiratory rate (RR) of patient. In some embodiments, the ventilation device 100 also includes a communication apparatus 190, which is configured to receive the blood oxygen parameter and the respiratory parameter of the patient which are transmitted externally. That is to say, in this embodiment, the ventilation device 100 does not obtain the blood oxygen parameter by itself and the respiratory parameter to be processed later, while other medical device(s) obtain(s) and transmit(s) these parameters to the ventilation device 100 for processing. In other embodiments, the sensor 140 and the communication apparatus 190 can be combined. For example, the ventilation device 100 obtains the respiratory parameter in real time through the sensor 140, and receives the externally transmitted blood oxygen parameter through the communication apparatus 190, and then processes the respiratory parameter and the blood oxygen parameter.

In this embodiment, the sensor 140 includes a pressure sensor which is arranged at the first signal acquisition point 132a and a flow sensor which is arranged at the second signal acquisition point 132b. For example, the flow sensor may be a hot-wire sensor or a pressure-difference sensor, etc. The pressure sensor is configured to acquire the pressure data at the first signal acquisition point 132a, which data can represent a pressure value at the first signal acquisition point 132a. When the ventilation device 100 delivers gas(es) at different gas flow rates, the pressure data also change accordingly. The flow sensor is configured to obtain the flow rate data at the second signal acquisition point 132b, which data can represent a flow rate value of the second signal acquisition point 132b. When the ventilation device 100 delivers gas(es) at different gas flow rates, the flow rate data also change accordingly. The first signal acquisition point 132a and the second signal acquisition point 132b are both positioned between the gas source interface 110 of the ventilation device 100 and the patient interface 131 of the ventilation device 100. Positions of the first signal acquisition point 132a and the second signal acquisition point 132b are illustrated in FIG. 1, but they do not represent actual positions of the first signal acquisition point 132a and the second signal acquisition point 132b. It can be understood that the first signal acquisition point 132a and the second signal acquisition point 132b can be arranged at any position in the ventilation device 100 where the flow rate data and the pressure data can be acquired. For example, the first signal acquisition point 132a and the second signal acquisition point 132b can be arranged in the flow rate delivery apparatus 120, specifically between the turbine (if any) and the respiratory pipeline 130, or inside the respiratory pipeline 130.

In some embodiments, the first signal acquisition point 132a and the second signal acquisition point 132b may also be the same signal acquisition point, that is, the pressure sensor and the flow sensor acquire data at the same signal acquisition point.

In some embodiments, the first signal acquisition point 132a and the second signal acquisition point 132b may be positioned inside the ventilation device 100. It can be understood that the ventilation device 100 typically has a housing for accommodating various functional components (not shown), and the first signal acquisition point 132a and the second signal acquisition point 132b are positioned inside the housing, which means that the pressure sensor and flow sensor are arranged inside the housing.

In some embodiments, the pressure data can be acquired through one or more pressure sensors, the flow rate data can be acquired through one or more flow sensors, and/or the pressure data and flow rate data can be measured through a same sensor.

In other embodiments, the pressure data may not be acquired through a pressure sensor, and/or the flow rate data may not be acquired through a flow sensor, and the processor 170 may use other methods to acquire the pressure data and the flow rate data. For example, the pressure data and the flow rate data can be estimated through a turbine parameter, such as a turbine speed.

The memory 150 can be configured to store data or program(s), such as data acquired by the sensor 140, data generated by the processor 170 through calculations, or image frame(s) generated by the processor 170 which image frame(s) can be 2D or 3D images; or the memory 150 can be configured to store a graphical user interface, one or more default image display settings, and programming instruction(s) for the processor 170. The memory 150 can be a tangible and non-transient computer-readable medium, such as flash memory, RAM, ROM, EEPROM, etc.

The ventilation device 100 can receive an inputted instruction signal through the human-machine interaction apparatus 160, which signal can include a signal for controlling a ventilation mode of the ventilation device 100, a signal for setting ventilation parameter(s), etc. The human-machine interaction apparatus 160 can include such as one or a combination of: a keyboard, a mouse, a scroll wheel, and a mobile input apparatus (such as a mobile apparatus with a touch screen, a mobile phone, etc.).

The processor 170 is configured to execute instructions or programs, control the flow rate delivery apparatus 120, the gas source interface 110 and/or various control valves inside the respiratory pipeline 130, or process the received data to generate a required calculation or determination result, or generate visual data or graphics, and output the visual data or graphics to the display apparatus 180 for displaying.

The display apparatus 180 is configured to display a ventilation interface, which displays at least one or all physiological parameters. The display apparatus 180 may include one or more display screens, and each display screen may be a touch screen or a non-touch screen. When the display apparatus 180 includes a touch screen, the touch screen can also be part of the human-machine interaction apparatus 160 for receiving user instruction(s) in response to user touch operation(s).

In this embodiment, a gas flow rate at which the ventilation device 100 provides respiratory support to the patient, or a theoretically output gas flow rate at which the ventilation device 100 provides respiratory support to the patient, is defined as a second gas flow rate. For example, medical staff set the gas flow rate to 40LPM on an operation panel of the oxygen therapy device, and the processor 170 controls the flow rate delivery apparatus 120 to provide oxygen therapy to patient at a second gas flow rate of 40LPM. The key to this embodiment lies in how to determine the second gas flow rate, or in other words, what to use as a reference to determine the second gas flow rate.

To determine the second gas flow rate, the processor 170 controls the flow rate delivery apparatus 120 to deliver gas(es) to patient interface 131 at multiple first gas flow rates, and calculates a characteristic parameter of flow rate for the patient according to the pressure data and the flow rate data under multiple first gas flow rates. The characteristic parameter of flow rate is related to a physiological state of the patient and can be used as a reference for determining the second gas flow rate.

The first gas flow rates mentioned above are also theoretical output gas flow rate of the ventilation device 100. For example, a current gas flow rate displayed on the ventilation interface is 40LPM, which means that the ventilation device 100 uses a second gas flow rate of 40LPM to perform oxygen therapy for the patient. Then, the ventilation device 100 delivers a gas to the patient at gas flow rates of 50LPM and 60LPM (the ventilation interface displays gas flow rates of 50LPM and 60LPM respectively), and calculates the characteristic parameter of flow rate According to the pressure data and the flow rate data under these two gas flow rates. In the above process, there are two first gas flow rates, namely 50LPM and 60LPM. In some embodiments, when the ventilation device provides respiratory support to the patient at a second gas flow rate, the processor 170 is further configured to, at a predetermined interval, repeatedly control the flow rate delivery apparatus 120 to deliver gas(es) to the patient interface 131 at the multiple first gas flow rates, and calculate the characteristic parameter of flow rate according to the pressure data and the flow rate data under the multiple first gas flow rates. That is to say, the ventilation device 100 periodically delivers gas(es) to the patient at multiple first gas flow rates to periodically calculate the characteristic parameter of flow rate.

For example, the second gas flow rate is 20LPM, three first gas flow rates which increase sequentially at an interval of 10LPM are preset, and a minimum first gas flow rate is preset to be 10LPM greater than the second gas flow rate, with a predetermined time length of 2 minutes. After providing respiratory support to the patient at a gas flow rate of 20LPM for 2 minutes, the ventilation device 100 sequentially delivers gas(es) to the patient at gas flow rates of 30LPM, 40LPM, and 50LPM. A time period for delivering the gas(es) to the patient according to one first gas flow rate can include one or more respiratory cycles. According to the pressure data and the flow rate data under the three first gas flow rates mentioned above, the characteristic parameter of flow rate can be calculated to be used as a reference for determining the second gas flow rate. Assuming that after obtaining a characteristic parameter of flow rate, the original second gas flow rate is changed from 20LPM to 30LPM according to this characteristic parameter of flow rate, the ventilation device 100 provides respiratory support to the patient at a gas flow rate of 30LPM for 2 minutes, and then delivers gas(es) to the patient sequentially at gas flow rates of 40LPM, 50LPM, and 60LPM, that is, entering a calculation cycle for a next characteristic parameter of flow rate.

The advantage of periodically using multiple first gas flow rates for the ventilation and calculation of the characteristic parameter of flow rate is that it can periodically provide a reference for determining the second gas flow rate.

It should be noted that the gas flow rate inhaled by a patient usually changes over time, that is, said gas flow rate is dynamic. For example, a physical condition of a patient may change at different times, and the gas flow rate inhaled may also change accordingly. The desired second gas flow rate for medical staff should approach or exceed the gas flow rate inhaled by the patient when calculating the characteristic parameter of flow rate. For example, in the above example, the original second gas flow rate is changed from 20LPM to 30LPM according to this characteristic parameter of flow rate, because 30LPM approaches or exceeds the gas flow rate inhaled by the patient when calculating this characteristic parameter of flow rate. However, technical personnel in this field can understand that due to a dynamic variation of the gas flow rate inhaled by the patient, the calculation process usually can only ensure that the second gas flow rate approaches or exceeds the gas flow rate inhaled by the patient when calculating the characteristic parameter of flow rate. In some embodiments, it may not necessarily guarantee that the second gas flow rate will always approach or exceed the gas flow rate inhaled by the patient. In addition, "when calculating this characteristic parameter of flow rate" should not be mechanically limited to execution time of "calculation" action. For example, in the above example, the ventilation device 100 periodically delivers gas(es) to the patient at multiple first gas flow rates, and each cycle calculates the characteristic parameter of flow rate once. The preset second gas flow rate approaches or exceeds the gas flow rate inhaled by the patient at any time in that cycle. In the next cycle, the gas flow rate inhaled by the patient may be higher than the second gas flow rate currently set or lower than the second gas flow rate currently set. Therefore, the second gas flow rate can be adjusted again, according to the characteristic parameter of flow rate calculated in the next cycle.

In some embodiments, the ventilation device 100 periodically delivers gas(es) to the patient at multiple first gas flow rates, and the multiple first gas flow rates do not change continuously or in a short period of time. After delivering gas(es) at a first gas flow rate, the ventilation device 100 can deliver gas(es) to the patient for a period of time at a third gas flow rate, and then change to deliver gas(es) to the patient at another first gas flow rate. For example, the second gas flow rate is 20LPM, three first gas flow rates are preset, wherein the first gas flow rate increases sequentially at intervals of 10LPM, and a minimum first gas flow rate is preset to be 10LPM greater than the third gas flow rate, with a predetermined time length of 2 minutes. Firstly, the ventilation device 100 delivers gas(es) to a patient at a flow rate of 30LPM for 2 minutes, and then provides respiratory support to the patient at a flow rate of 20LPM for 2 minutes. Then, the ventilation device 100 delivers gas(es) to the patient at a flow rate of 40LPM for 2 minutes, and then continues to provide respiratory support to the patient at a flow rate of 20LPM for 2 minutes. Then, the ventilation device 100 delivers gas(es) to the patient at a flow rate of 50LPM for 2 minutes, and then continues to provide respiratory support to the patient at a flow rate of 20LPM. That is to say, in the process of delivering gas(es) to the patient at multiple first gas flow rates to calculate the characteristic parameter of flow rate, the process of ventilation with other flow rate(s), which is(are) unrelated to the calculation of characteristic parameter of flow rate can be inserted, as long as it does not interfere with the delivery of gas to the patient at the multiple first gas flow rates to calculate the characteristic parameter of flow rate.

In some embodiments, the ventilation device 100 is triggered by a flow rate to deliver gas(es) to the patient at multiple first gas flow rates. Specifically, when the flow rate delivery apparatus 120 delivers gas(es) to the patient interface 131 at the second gas flow rate to provide respiratory support to the patient, the processor 170 compares the flow rate data obtained from the flow sensor with a fourth threshold range. After identifying that the flow rate data exceeds the fourth threshold range, the processor 170 controls the flow rate delivery apparatus 120 to deliver gas(es) to the patient interface 131 at multiple first gas flow rates, and calculates the characteristic parameter of flow rate according to the pressure data and the flow rate data under the multiple first gas flow rates.

For example, the second gas flow rate is 20LPM, three first gas flow rates, which increase sequentially at intervals of 10LPM, are preset. A minimum first gas flow rate is preset to be 10LPM greater than the second gas flow rate. During providing respiratory support to patient at a gas flow rate of 20LPM in the ventilation device 100, the processor 170 compares the flow rate data with the fourth threshold range. If the flow rate data exceeds the fourth threshold range, it means that an actual gas flow generated under the second gas flow rate which is currently preset, does not satisfy expectations. The ventilation device 100 delivers gas(es) to the patient sequentially at gas flow rates of 30LPM, 40LPM, and 50LPM. A time period for delivering gas(es) to the patient according to a first gas flow rate can include one or more respiratory cycles. According to the pressure data and the flow rate data of the three first gas flow rates mentioned above, the characteristic parameter of flow rate can be calculated to be used as a reference for determining the second gas flow rate. For example, the second gas flow rate can be reset according to the characteristic parameter of flow rate to stabilize the flow rate data within the fourth threshold range without being too high or too low. In some embodiments, the fourth threshold range may be updated with an update of the second gas flow rate. In other embodiments, the fourth threshold range may also be a fixed range.

The advantage of delivering gas(es) to the patient at multiple first gas flow rates based on a triggering of flow rate mentioned above is that the second gas flow rate can be adjusted or preset for the purpose of stabilizing the flow rate, so as to ensure that the actual gas flow rate generated under the second gas flow rate satisfies expectations.

In some embodiments, the ventilation device 100 is triggered by a pressure to deliver gas(es) to the patient at multiple first gas flow rates. Specifically, when the flow rate delivery apparatus 120 delivers gas(es) to the patient interface 131 at the second gas flow rate to provide respiratory support to the patient, the processor 170 compares the pressure data obtained from the pressure sensor with a fifth threshold range. After identifying that the pressure data exceeds the fifth threshold range, the processor 170 controls the flow rate delivery apparatus 120 to deliver gas(es) to the patient interface 131 at multiple first gas flow rates, and calculates the characteristic parameter of flow rate according to the pressure data and the flow rate data under the multiple first gas flow rates.

For example, the second gas flow rate is 20LPM, three first gas flow rates, which increase sequentially at intervals of 10LPM, are preset. A minimum first gas flow rate is preset to be 10LPM greater than the second gas flow rate. During providing respiratory support to patient at a gas flow rate of 20LPM in the ventilation device 100, the processor 170 compares the pressure data with the fifth threshold range. If the pressure data exceeds the fifth threshold range, it means that an actual gas pressure generated under the second gas flow rate which is currently preset, does not meet expectations. The ventilation device 100 delivers gas(es) to the patient sequentially at gas flow rates of 30LPM, 40LPM, and 50LPM. A time period for delivering gas(es) to the patient according to a first gas flow rate can include one or more respiratory cycles. According to the pressure data and the flow rate data of the three first gas flow rates mentioned above, the characteristic parameter of flow rate can be calculated as a reference for determining the second gas flow rate. For example, the second gas flow rate can be reset according to the characteristic parameter of flow rate to stabilize the pressure data within the fifth threshold range without being too high or too low. In some embodiments, the fifth threshold range may be updated with an update of the second gas flow rate. In other embodiments, the fifth threshold range may also be a fixed range.

The advantage of delivering gas(es) to the patient at multiple first gas flow rates based on a triggering of pressure mentioned above is that the second gas flow rate can be adjusted or preset for the purpose of stabilizing the pressure, so as to ensure that the actual gas pressure generated under the second gas flow rate satisfies expectations.

In some embodiments, the ventilation device 100 has two ventilation phases, wherein the processor 170 controls the ventilation device 100 to deliver gas(es) to the patient interface 131 at multiple first gas flow rates during the first ventilation phase, and calculates a characteristic parameter of flow rate for the patient according to the pressure data and the flow rate data under the multiple first gas flow rates. The processor 170 further controls the flow rate delivery apparatus 120 to deliver gas(es) to the patient interface 131 at the second gas flow rate, so as to provide respiratory support to the patient.

For example, after the ventilation device 100 starts providing respiratory support, it first enters a first ventilation phase, and then enters a second ventilation phase. Moreover, the first ventilation phase has three first gas flow rates, namely 20LPM, 30LPM, and 40LPM, by default. In the first ventilation phase, the ventilation device 100 delivers gas(es) to the patient sequentially at gas flow rates of 20LPM, 30LPM, and 40LPM. A time period for delivering the gas to the patient according to one first gas flow rate can include one or more respiratory cycles. According to the pressure data and the flow rate data under the three first gas flow rates mentioned above, the characteristic parameter of flow rate can be calculated as a reference for determining the second gas flow rate in the second ventilation phase. For example, after calculating the characteristic parameter of flow rate, the second gas flow rate in the second ventilation phase can be set to 30LPM. From this embodiment, it can also be seen that the second gas flow rate and one of the multiple first gas flow rates can also be the same.

From the above description, it can be seen that during providing respiratory support, the ventilation device 100 can smoothly transition to provide the multiple first gas flow rates. Patient does not need to remove a mask or nasal catheter, nor does the ventilation device 100 need to temporarily stop delivering gas(es) to the patient. The characteristic parameter of flow rate can be determined as a reference, thereby minimizing influence(s) of other factor(s) on determining the gas flow rate that the patient needs to inhale.

The following continues to explain how to calculate the characteristic parameter of flow rate for the patient according to the pressure data and the flow rate data under the multiple first gas flow rates.

In some embodiments, during delivering gas(es) to the patient at each first gas flow rate by the ventilation device 100, the processor 170 calculates a proximal gas characteristic of a proximal end of the patient in a period of time under each first gas flow rate, according to the pressure data and the flow rate data in said period of time under each first gas flow rate, and then determines the characteristic parameter of flow rate according to the proximal gas characteristics. Wherein, the period of time can be a total time length of ventilation under one first gas flow. For example, if gas(es) is(are) to the patient at a first gas flow rate during i-th respiratory cycle of the patient, a proximal gas characteristic of the proximal end of the patient during the i-th respiratory cycle can be calculated according to the pressure data and the flow rate data during the i-th respiratory cycle. The period of time can also be a part of the total time length of ventilation under a first gas flow rate. For example, if gas(es) is(are) delivered to the patient at a first gas flow rate during i-th respiratory cycle of the patient, a proximal gas characteristic of the patient during a part of the i-th respiratory cycle can be calculated according to the pressure data and the flow rate data during said part of the i-th respiratory cycle.

In the description of the characteristic parameter of flow rate determined above, the proximal gas characteristic of the proximal end of the patient refer to a gas characteristic at a position which is close to the patient interface 131. The proximal end here should not be mechanically understood. For technical personnel in this field, a proximal end and a distal end of the patient are relative concepts, and a position relatively close to the patient interface 131 can be understood as the proximal end of the patient. For example, when the ventilation device 100 is an oxygen therapy device, the oxygen therapy device itself can used as a distal end of the patient. A pipeline between the oxygen therapy device and the nasal catheter, as well as the nasal catheter itself, can be understood as the proximal end of the patient, as long as it is closer to the first signal acquisition point 132a and the second signal acquisition point 132b. The gas characteristic refers to characteristic or feature that can reflect a flow rate and/or a pressure. From this, it can be seen that the process of delivering gas(es) to the patient at multiple first gas flow rates, so as to determine the characteristic parameter of flow rate, is actually to calculate a characteristic or a feature of a flow rate and/or a pressure for a proximal end of a patient through the actual pressure data and the actual flow rate data when delivering gas(es) to the patient at each first gas flow rate, and then determine the characteristic parameter of flow rate according to the characteristic or the feature of the flow rate and/or the pressure for the proximal end of the patient.

It should be noted that the characteristic or feature of the flow rate and/or the proximal pressure of the patient is/are calculated according to the pressure data at the first signal acquisition point 132a and the flow rate data at the second signal acquisition point 132b, rather than directly measured at the proximal end of the patient. For example, when the proximal gas characteristic is a proximal pressure of the patient, it is not necessary to arrange another pressure sensor at the proximal end of the patient to measure the proximal pressure. This is because the inventor found that there are many problems with arranging another pressure sensor at the proximal end of the patient to measure the proximal pressure. Firstly, there is not enough space near the patient interface 131 to install another pressure sensor. Secondly, the closer the ventilation device 100 to the patient interface 131, the greater the impact on the measurement accuracy of the sensor. Thirdly, some ventilation devices 100 often replace the pipeline near the patient interface 131, and installing a pressure sensor would result in higher costs.

Taking the proximal gas characteristic as the proximal pressure for example, we continue to explain how the technical solution of this disclosure calculates the proximal pressure according to the pressure data and the flow rate data.

In some embodiments, a model is first established to describe a relationship between pressure difference(s) and the flow rate data. Pressure difference refers to a pressure difference between the first signal acquisition point 132a and the proximal end of the patient. This model can be a mathematical expression, a curve, a table lookup, or a machine learning model. The inventor found that the pressure difference mentioned above is related to a type and a model of the respiratory pipeline (ventilation loop) and of the nasal catheter, and is not related to an outlet gap of the patient interface 131. Due to differences in resistance characteristic between different types and models of ventilation pipelines and nasal catheters, it is necessary to perform an operation to obtain the above function relationship after replacing the nasal catheter or other operations. Specifically, the gas flow rate preset by the ventilation device 100 can be gradually changed, and the pressure data and the flow rate data under different gas flow rates can be obtained, as well as pressure values at the proximal end of the patient can be measured. Under each flow rate data, one pressure difference can be obtained, and then the relationship between the flow rate data and pressure difference can be expressed through curve fitting, as shown in FIG. 2. From the above description, it can be seen that establishing a model is somewhat similar to obtaining the characteristic parameter of flow rate, both of which require gas(es) to be delivered outward at different flow rates. However, the two are fundamentally different. When establishing a model, there is no need to connect the respiratory system of a patient. Therefore, an external pressure sensor can be configured to measure the pressure value at the proximal end of the patient. For example, a component with a pressure sensor at one end can be inserted into the patient interface 131 to measure the pressure near the patient interface 131. In some embodiments, the above model may be imported by external devices. In other embodiments, the above model can also be obtained through ventilation experiments on the ventilation device 100.

In some embodiments, the preset model can be expressed as ΔP = f (Flow), for example ΔP = *K,* * *Flow;* wherein Flow is the flow rate data obtained, K1 is a coefficient obtained by fitting the pressure difference and the flow rate data, then *P*ₙ = *Press* - ΔP = Press - K, * *Flow* is obtained, wherein Pn is the proximal pressure, and Press is the pressure data. By inputting the obtained pressure data and flow rate data into this equation, the proximal pressure can be calculated. As shown in FIG. 3, curves of pressure data, flow rate data, and proximal pressure over time under a first gas flow rate show that the patient has undergone multiple respiratory cycles under this first gas flow rate. From this graph, it can be seen that along with respiration of the patient, the pressure data and the flow rate data change with time, the calculated proximal pressure also changes accordingly. In addition to the above expressions, other forms of polynomials, exponential functions, logarithmic functions, and power functions can also be configured to represent the model. In some embodiments, the obtained model can also be stored in advance in the ventilation device 100, and the corresponding model can be automatically matched by the user, through setting on the operation interface, information, such as the type and model of the pipeline and nasal catheter.

The above method for calculating the proximal pressure can reduce the interference of gas leakage at the patient interface 131, making the obtained proximal pressure more accurate.

From the above description, it can be seen that the proximal gas characteristic is a value that changes over time. In some embodiments, based on the proximal gas characteristic that changes over time, a gas characteristic value corresponding to each first gas flow rate can be obtained. That is, one first gas flow rate has one gas characteristic value. After calculating the gas characteristic value under each first gas flow rate, the processor 170 takes the first gas flow rate, which corresponds to a gas characteristic value that satisfies a preset condition, as the characteristic parameter of flow rate. That is to say, the ventilation device 100 delivers gas(es) at multiple first gas flow rates and obtains the corresponding gas characteristic values. If there is a gas characteristic value that satisfies a preset condition under a certain first gas flow rate, the first gas flow rate is used as a reference for determining the second gas flow rate. Then we continue to use the proximal pressure as an example of the proximal gas characteristic to illustrate. It can be understood that an inspiratory flow rate of the patient changes during inhalation, which leads to a change in the proximal pressure. When delivering gas(es) to the patient interface 131 at the same gas flow rate, the proximal pressure decreases when the gas flow rate inhaled by the patient increases; the proximal pressure increases when the gas flow rate inhaled by the patient decreases.

In some embodiments, the proximal pressures under each first gas flow rate are statistically analyzed to obtain a pressure statistical value. The pressure statistical value is used as the gas characteristic value, and the pressure statistical value is any one of a mean value, a standard deviation value, a median value, a maximum value, and a minimum value. Then, whether the pressure statistical value falls into a preset first threshold range is determined. If the pressure statistical value falls into the first threshold range, it indicates that the first gas flow rate corresponding to the pressure statistical value can be used as a reference for determining the second gas flow rate.

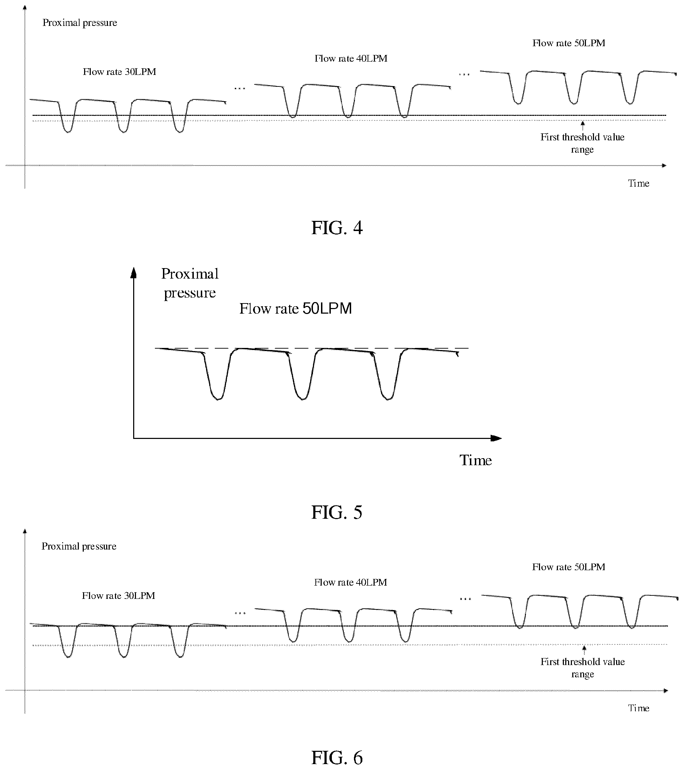

For example, in FIG. 4, the first threshold range is shown by a dashed line, and the minimum value of the proximal pressure is used as the pressure statistical value. When the first gas flow rate is 40LPM, the minimum value of the proximal pressure falls into the first threshold range, so the characteristic parameter of flow rate is 40LPM.

During any one respiratory cycle, when the gas flow rate inhaled by the patient at the proximal end is the highest, the proximal pressure reaches the minimum value. Therefore, when the proximal pressure reaches the minimum value, it means that the current gas flow rate inhaled by the patient is the peak inspiratory flow rate. If the minimum value of the proximal pressure is within the first threshold range, it indicates that the gas flow rate, which can be provided by the proximal end of the patient under the current first gas flow rate, approaches or exceeds the inspiratory peak flow rate of the patient. Therefore, this first gas flow rate can be used as a reference for determining the second gas flow rate. For example, the second gas flow rate should not be less than the current first gas flow rate. The first threshold range can be determined based on the clinical experience of medical staff or other physiological parameters of the patient. For example, if the first threshold range is a fixed value of 0, it means that when delivering gas(es) at multiple first gas flow rates, a first gas flow rate must be found, at which first gas flow rate, the proximal end of the patient will not generate negative pressure, that is, a gas flow rate at the proximal end of the patient is > gas leakage +peak inspiratory flow rate. This equation is also easy to understand. If the gas flow rate at the proximal end of the patient is<gas leakage+peak inspiratory flow rate, the patient not only needs to draw a gas from the respiratory pipeline, but also inhales a gas from the outside, resulting in a proximal pressure less than 0. The first threshold range can also be obtained based on positive end respiratory pressure, for example, the first threshold range is between the positive end respiratory pressure of the patient to a sum of the positive end expiratory pressure and a constant.

In some embodiments, when delivering gas(es) at any first gas flow rate, a first time length, in which gas(es) is(are)s delivered to the patient interface 131 at the first gas flow rate, is recorded, and a second time length within the first time length, in which second time length the proximal pressure is greater than a preset pressure threshold, is also recorded. Take a ratio of the first time length to the second time length as the gas characteristic value of the first gas flow rate. If the ratio falls into a preset second threshold range, it indicates that a time length, in which the proximal pressure stabilizes within a certain range, is expected, and thus indicates that a time length, in which, the gas flow rate at the proximal end of the patient stabilizes within a certain range, is expected. For example, a time length, in which, the gas flow rate approaches or exceeds the inspiratory peak flow rate of the patient, is expected. Therefore, the corresponding first gas flow rate can be used as a reference for determining the second gas flow rate.

In some embodiments, when delivering gas(es) at any first gas flow rate, an integral value of the proximal pressure over time during the inspiratory phase of the patient is obtained, and the integral value is used as the gas characteristic value. The curve in FIG. 5 shows variation(s) of proximal pressure over time under a first gas flow rate, wherein the dashed line is a baseline. An area formed by the variation curve of proximal pressure and the baseline is the integral value of the proximal pressure over time. This integral value can represent work done by the patient during the inspiratory phase. The baseline can be preset by medical staff based on the condition of the patient, for example, the baseline is generated from the calculated proximal pressure when the ventilation device 100 has not started ventilation and the patient has not started respiration after being connected to the patient interface 131. At this time, the baseline represents the proximal pressure, when the patient has not yet started respiration and the ventilation device 100 has not yet started ventilation. If the integral value falls into a preset third threshold range, it indicates that the work done by the patient during the inspiratory phase is within a reasonable range. Therefore, the first gas flow rate corresponding to this integral value can be used as a reference for determining the second gas flow rate. Of course, the baseline can also be automatically preset by the ventilation device 100.

In the above embodiments, a first gas flow rate, which corresponds with a gas characteristic value that satisfies the preset condition, is used as the characteristic parameter of flow rate. In other embodiments, the characteristic parameter of flow rate may not necessarily correspond exactly to one first gas flow rate. At this time, the first gas flow rate may correspond with more than one gas characteristic values that satisfy the preset condition. Therefore, the characteristic parameter of flow rate can be calculated based on a first gas flow rate which corresponds to multiple gas characteristic values that satisfy the preset condition. For example, if gas characteristic value(s), which correspond(s) to n first gas flow rates, all satisfies(satisfy) present condition(s), various fitting methods can be used to obtain one gas flow rate as the characteristic parameter of flow rate, based on these n first gas flow rates.

For example, in FIG. 6, the first threshold range is the dashed part, and the minimum value of the proximal pressure is used as a pressure statistical value. When the first gas flow rate is 40LPM and the first gas flow rate is 50LPM, a minimum value of the proximal pressures falls into the first threshold range. Therefore, an average value of the two first gas flow rates can be used as the characteristic parameter of flow rate, or the two first gas flow rates can be multiplied by a preset coefficient and added together to obtain the characteristic parameter of flow rate.

In some embodiments, after obtaining the characteristic parameter of flow rate, the ventilation device 100 automatically determines whether the second gas flow rate needs to be reset, and sets the second gas flow rate according to the characteristic parameter of flow rate, when the second gas flow rate is determined as needing to be reset. That is, obtaining the characteristic parameter of flow rate does not necessarily mean resetting the second gas flow rate. For example, if the current second gas flow rate is 30LPM, then the gas characteristic value under 30LPM is calculated. Then, gas(es) is(are) at the first gas flow rates of 40LPM and 50LPM, respectively; and the gas characteristic values under 40LPM and 50LPM are calculated. Finally, after determination, it is found that the gas characteristic value under 30LPM satisfies the preset condition. Therefore, the current second gas flow rate does not need to be reset. In this case, the second gas flow rate is equal to one of multiple first gas flow rates.

In some embodiments, after obtaining a new characteristic parameter of flow rate, the processor 170 compares the newly obtained current characteristic parameter of flow rate with a previously obtained characteristic parameter of flow rate, and determines that the second gas flow rate needs to be reset, when the characteristic parameter of flow rate changes. For example, when the ventilation device 100 regularly delivers gas(es) at multiple first gas flow rates, if the characteristic parameter of flow rate obtained in the next cycle are the same as that obtained in the previous cycle(s), it means that the reference for setting the second gas flow rate has not changed, and naturally there is no need to reset the second gas flow rate.

In some embodiments, the mode of setting the second gas flow rate according to the characteristic parameter of flow rate includes: obtaining a change value of flow rate, taking a sum of the characteristic parameter of flow rate and the change value of flow rate as the second gas flow rate, wherein the change value of flow rate can be a preset constant, and can be positive or 0. For example, if a current second gas flow rate is 30LPM and the change value of flow rate is SLPM, and the characteristic parameter of flow rate calculated after delivering gas(es) at multiple first gas flow rates is 35LPM, then the processor 170 controls a gas flow rate at which the flow rate delivery apparatus deliver gas(es)120 to change from 30LPM to 40LPM.

In some embodiments, the mode of setting the second gas flow rate according to the characteristic parameter of flow rate includes: obtaining a proportional parameter, multiplying the characteristic parameter of flow rate by the proportional parameter, and using the product as the second gas flow rate. Wherein, the proportion parameter can be a preset constant, and can be greater than or equal to 1. For example, if the current second gas flow rate is 20LPM and the proportional parameter is 1.2, and the characteristic parameter of flow rate calculated after delivering gas(es) at multiple first gas flow rates is 25LPM, then the processor 170 controls a gas flow rate at which the flow rate delivery apparatus deliver gas(es)120 to change from 20LPM to 30LPM.

In addition to automatically setting the second gas flow rate, a power to reset the second gas flow rate can also be delegated to the user.

In some embodiments, after obtaining the characteristic parameter of flow rate, the characteristic parameter of flow rate is displayed on the ventilation interface. The user can consider whether to reset the second gas flow rate according to the displayed characteristic parameter of flow rate. If it is necessary to reset the second gas flow rate, the characteristic parameter of flow rate can also be used as a reference to determine whether to increase or decrease the second gas flow rate. In this process, the user can also make decisions, combining other physiological parameters obtained. In some embodiments, the characteristic parameter of flow rate can also be compared with a preset flow threshold range. If the characteristic parameter of flow rate exceeds the flow threshold range, an alarm is issued. For example, when the characteristic parameter of flow rate exceed an upper limit of the flow threshold range, the user is indicated that the inspiratory peak flow rate of the patient is too high, and the current set flow rate cannot satisfies inspiratory requirements of the patient.

In some embodiments, information, which is related to the characteristic parameter of flow rate, is outputted to indicate to the user to set the second gas flow rate. For example, if the obtained characteristic parameter of flow rate is higher than the current second gas flow rate, the user is indicated to increase the second gas flow rate appropriately. If the obtained characteristic parameter of flow rate is lower than the current second gas flow rate, the user is indicated to decrease the second gas flow rate appropriately. In addition, in addition to the regulation direction, indication for an regulation amount, can also be provided. For example, when indicating the user to increase the current second gas flow rate, a suggestion for how much to increase can also be given. When indicating the user to decrease the current second gas flow rate, a suggestion for how much to decrease can also be given. For example, a dialog box can pop up, and when the user clicks accept, they can reset the second gas flow rate according to the suggestion in the dialog box. If the user does not accept it, the current second gas flow rate can be maintained to continue the ventilation.

In some embodiments, after obtaining the characteristic parameter of flow rate, the processor 170 compares the current characteristic parameter of flow rate with the previous obtained characteristic parameter of flow rate. If the current characteristic parameter of flow rate changes when compared to the previous obtained characteristic parameter of flow rate, information related to the changed characteristic parameter of flow rate is outputted to indicate to the user to increase or decrease the second gas flow rate. For example, when the ventilation device 100 regularly delivers gas(es) at multiple first gas flow rates, if the characteristic parameter of flow rate obtained in the next cycle increases, when compared to the characteristic parameter of flow rate obtained in the previous cycle, the user is indicated to increase the second gas flow rate.

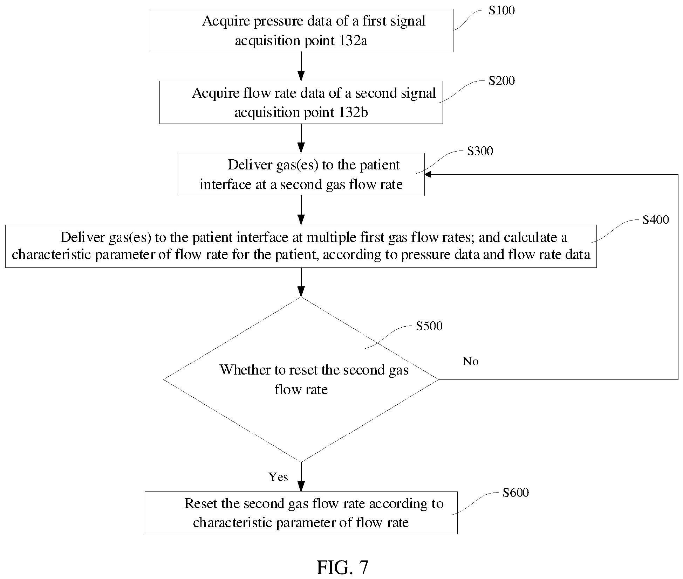

Please refer to the embodiment shown in FIG. 7, which provides a method for regulating a gas flow rate of a ventilation device, including:
Step S100, acquire pressure data of a first signal acquisition point 132a.
Step S200, acquire flow rate data of a second signal acquisition point 132b.

Wherein, the first signal acquisition point 132a and the second signal acquisition point 132b are both positioned between the gas source interface of the ventilation device and the patient interface 131 of the ventilation device. The gas source interface is capable of being connected with the gas source, and the patient interface 131 is capable of being connected with a respiratory system of a patient. The first signal acquisition point 132a and the second signal acquisition point 132b can be the same or different. For example, the first signal acquisition point 132a and the second signal acquisition point 132b can be positioned inside the ventilation device 100 itself.

Step S300, deliver gas(es) to the patient interface 131 at a second gas flow rate, so as to provide respiratory support to the patient, wherein the second gas flow rate approaches or exceeds a gas flow rate which is inhaled by the patient, when calculating a characteristic parameter of flow rate for the patient.

It can be understood that the desired second gas flow rate for medical staff should approach or exceed the gas flow rate inhaled by the patient when calculating the characteristic parameter of flow rate for the patient, so as to satisfy inspiratory requirement of the patient as much as possible.

Step S400, deliver gas(es) to the patient interface 131 at multiple first gas flow rates; and calculate the characteristic parameter of flow rate, according to the pressure data and the flow rate data under the multiple first gas flow rates.

The characteristic parameter of flow rate is related to a physiological state of the patient and can be used as a reference to determine the second gas flow rate in step S300. The first gas flow rate here is theoretical output gas flow rate of the ventilation device 100. For example, a current gas flow rate displayed on the ventilation interface is 40LPM, that is, the ventilation device 100 provides oxygen therapy to the patient at a second gas flow rate of 40LPM. Then, the ventilation device 100 respectively delivers gas(es) to the patient at a gas flow rate of 50LPM and of 60LPM (the ventilation interface displays gas flow rate of 50LPM and of 60LPM respectively), and the characteristic parameter of flow rate is calculated according to the pressure data and the flow rate data under these two gas flow rates. Therefore, there are two first gas flow rates in the above process, namely 50LPM and 60LPM, respectively.

In some embodiments, gas(es) is(are) periodically delivered to the patient at multiple first gas flow rates to periodically calculate the characteristic parameter of flow rate. Therefore, step S400 is repeated every predetermined time length to obtain characteristic parameter of flow rates.

For example, the second gas flow rate is 20LPM, three first gas flow rates, which increase sequentially at intervals of 10 LPM are preset, and a minimum first gas flow rate is preset to be 10 LPM greater than the second gas flow rate, with a predetermined time length of 2 minutes. After providing respiratory support to the patient at a gas flow rate of 20LPM for 2 minutes, the ventilation device 100 sequentially delivers gas(es) to the patient at gas flow rates of 30LPM, 40LPM, and 50LPM. A time period for delivering gas(es) to the patient according to one first gas flow rate can include one or more respiratory cycles. According to the pressure data and the flow rate data under the three first gas flow rates mentioned above, the characteristic parameter of flow rate can be calculated to be used as a reference for determining the second gas flow rate. Assuming that after obtaining a characteristic parameter of flow rate, the original second gas flow rate is changed from 20LPM to 30LPM according to this characteristic parameter of flow rate, the ventilation device 100 provides respiratory support to the patient at a gas flow rate of 30LPM for 2 minutes, and then delivers gas(es) to the patient sequentially at a gas flow rate of 40LPM, 50LPM, and 60LPM, that is entering a calculation cycle of a next characteristic parameter of flow rate.

In some embodiments, step S400 is triggered by a flow rate. Specifically, when delivering gas(es) to patient interface 131 at the second gas flow rate, so as to provide respiratory support to the patient, the obtained flow rate data are compared with a fourth threshold range. After identifying that the flow rate data exceeds the fourth threshold range, step S400 is executed. In some embodiments, the fourth threshold range may be updated with an update of the second gas flow rate. In other embodiments, the fourth threshold range may also be a fixed range.

In some embodiments, step S400 is triggered by a pressure. Specifically, when delivering gas(es) to patient interface 131 at the second gas flow rate to provide respiratory support to the patient, the obtained pressure data are compared with a fifth threshold range. After identifying that the pressure data exceeds the fifth threshold range, step S400 is executed. In some embodiments, the fifth threshold range may be updated with an update of the second gas flow rate. In other embodiments, the fifth threshold range may also be a fixed range.

In some embodiments, the ventilation device 100 has two ventilation phases. In the first ventilation phase, the ventilation device 100 delivers gas(es) to the patient interface 131 at multiple first gas flow rates, and calculates a characteristic parameter of flow rate for the patient according to the pressure data and the flow rate data under the multiple first gas flow rates. In the second ventilation phase, the ventilation device 100 delivers gas(es) to the patient interface 131 at the second gas flow rate, so as to provide respiratory support to the patient. The second gas flow rate in the second ventilation phase can be determined according to the characteristic parameter of flow rate in the first ventilation phase. That is to say, first execute step S400 to calculate the characteristic parameter of flow rate, and then execute step S300 according to the calculated characteristic parameter of flow rate.

For example, after the ventilation device 100 starts providing respiratory support, it first enters a first ventilation phase, and then enters a second ventilation phase. Moreover, the first ventilation phase has three first gas flow rates, namely 20LPM, 30LPM, and 40LPM, by default. In the first ventilation phase, the ventilation device 100 delivers gas(es) to the patient sequentially at gas flow rates of 20LPM, 30LPM, and 40LPM. A time period for delivering the gas to the patient according to one first gas flow rate can include one or more respiratory cycles. According to the pressure data and the flow rate data under the three first gas flow rates mentioned above, the characteristic parameter of flow rate can be calculated as a reference for determining the second gas flow rate in the second ventilation phase. For example, after calculating the characteristic parameter of flow rate, the second gas flow rate in the second ventilation phase can be set to 30LPM. From this embodiment, it can also be seen that the second gas flow rate and one of the multiple first gas flow rates can also be the same.

The following continues to explain how to calculate the characteristic parameter of flow rate of patient according to the pressure data and the flow rate data under the multiple first gas flow rates.

In some embodiments, during delivering gas(es) to the patient at each first gas flow rate by the ventilation device 100, a proximal gas characteristic of a proximal end of the patient in a period of time under each first gas flow rate, is calculated according to the pressure data and the flow rate data in said period of time under each first gas flow rate; then the characteristic parameter of flow rate is determined according to the proximal gas characteristics. Wherein, the period of time can be a total time length of ventilation under one first gas flow. For example, if gas(es) is(are) to the patient at a first gas flow rate during i-th respiratory cycle of the patient, a proximal gas characteristic of the proximal end of the patient during the i-th respiratory cycle can be calculated according to the pressure data and the flow rate data during the i-th respiratory cycle. The period of time can also be a part of the total time length of ventilation under a first gas flow rate. For example, if gas(es) is(are) delivered to the patient at a first gas flow rate during i-th respiratory cycle of the patient, a proximal gas characteristic of the patient during a part of the i-th respiratory cycle can be calculated according to the pressure data and the flow rate data during said part of the i-th respiratory cycle.

In the description of the characteristic parameter of flow rate determined above, the proximal gas characteristic of the proximal end of the patient refer to a gas characteristic at a position which is close to the patient interface 131. The proximal end here should not be mechanically understood. For technical personnel in this field, a proximal end and a distal end of the patient are relative concepts, and a position relatively close to the patient interface 131 can be understood as the proximal end of the patient. The gas characteristic refers to characteristic or feature that can reflect a flow rate and/or a pressure. From this, it can be seen that the process of delivering gas(es) to the patient at multiple first gas flow rates, so as to determine the characteristic parameter of flow rate, is actually to calculate a characteristic or a feature of a flow rate and/or a pressure for a proximal end of a patient through the actual pressure data and the actual flow rate data when delivering gas(es) to the patient at each first gas flow rate, and then determine the characteristic parameter of flow rate according to the characteristic or the feature of the flow rate and/or the pressure for the proximal end of the patient.

Taking the proximal gas characteristic as the proximal pressure for example, we continue to explain how the technical solution of this disclosure calculates the proximal pressure according to the pressure data and the flow rate data.

In some embodiments, a model is first established to describe a relationship between pressure difference(s) and the flow rate data. Pressure difference refers to a pressure difference between the first signal acquisition point 132a and the proximal end of the patient. This model can be a mathematical expression, a curve, a table lookup, or a machine learning model. The inventor found that the pressure difference mentioned above is related to a type and a model of the respiratory pipeline (ventilation loop) and of the nasal catheter, and is not related to an outlet gap of the patient interface 131. Due to differences in resistance characteristic between different types and models of ventilation pipelines and nasal catheters, it is necessary to perform an operation to obtain the above function relationship after replacing the nasal catheter or other operations. Specifically, the gas flow rate preset by the ventilation device 100 can be gradually changed, and the pressure data and the flow rate data under different gas flow rates can be obtained, as well as pressure values at the proximal end of the patient can be measured. Under each flow rate data, a pressure difference can be obtained, and then the relationship between the flow rate data and pressure difference can be expressed through curve fitting, as shown in FIG. 2. From the above description, it can be seen that establishing a model is somewhat similar to obtaining the characteristic parameter of flow rate, both of which require gas to be delivered outward at different flow rates. However, the two are fundamentally different. When establishing a model, there is no need to connect the respiratory system of a patient. Therefore, an external pressure sensor can be configured to measure the pressure value at the proximal end of the patient. For example, a component with a pressure sensor at one end can be inserted into the patient interface 131 to measure the pressure near the patient interface 131. In some embodiments, the above model may be imported by external devices. In other embodiments, the above model can also be obtained through ventilation experiments on the ventilation device 100.

In some embodiments, the preset model can be expressed as ΔP = f (Flow), for example ΔP = *K*₁ * *Flow;* wherein Flow is the flow rate data obtained, K1 is a coefficient obtained by fitting the pressure difference and the flow rate data, then *P*ₙ = *Press* - ΔP = Press - *K*₁ * *Flow* is obtained, wherein Pn is the proximal pressure, and Press is the pressure data. By inputting the obtained pressure data and flow rate data into this equation, the proximal pressure can be calculated. As shown in FIG. 3, curves of pressure data, flow rate data, and proximal pressure over time under a first gas flow rate show that the patient has undergone multiple respiratory cycles under this first gas flow rate. From this graph, it can be seen that along with respiration of the patient, the pressure data and the flow rate data change with time, the calculated proximal pressure also changes accordingly. In addition to the above expressions, other forms of polynomials, exponential functions, logarithmic functions, and power functions can also be configured to represent the model. In some embodiments, the obtained model can also be stored in advance in the ventilation device 100, and the corresponding model can be automatically matched by the user, through setting on the operation interface, information, such as the type and model of the pipeline and nasal catheter.

From the above description, it can be seen that the proximal gas characteristic is a value that changes over time. In some embodiments, based on the proximal gas characteristic that changes over time, gas characteristic values corresponding to each first gas flow rate can be obtained. That is, one first gas flow rate has one gas characteristic value. After calculating the gas characteristic value under each first gas flow rate, the first gas flow rate, which corresponds to a gas characteristic value that satisfies a preset condition, is taken as the characteristic parameter of flow rate. That is to say, the ventilation device 100 delivers gas(es) at multiple first gas flow rates and obtains the corresponding gas characteristic values. If there is a gas characteristic value that satisfies a preset condition under a certain first gas flow rate, the first gas flow rate is used as a reference for determining the second gas flow rate. Then we continue to use the proximal pressure as an example of the proximal gas characteristic to illustrate. It can be understood that an inspiratory flow rate of the patient changes during inhalation, which leads to a change in the proximal pressure. When delivering gas(es) to the patient interface 131 at the same gas flow rate, the proximal pressure decreases when the gas flow rate inhaled by the patient increases; the proximal pressure increases when the gas flow rate inhaled by the patient decreases.

In some embodiments, the proximal pressures under each first gas flow rate are statistically analyzed to obtain a pressure statistical value. The pressure statistical value is used as the gas characteristic value, and the pressure statistical value is any one of a mean value, a standard deviation value, a median value, a maximum value, and a minimum value. Then, whether the pressure statistical value falls into a preset first threshold range is determined. If the pressure statistical value falls into the first threshold range, it indicates that the first gas flow rate corresponding to the pressure statistical value can be used as a reference for determining the second gas flow rate.

For example, in FIG. 4, the first threshold range is shown by a dashed line, and the minimum value of the proximal pressure is used as the pressure statistical value. When the first gas flow rate is 40LPM, the minimum value of the proximal pressure falls into the first threshold range, so the characteristic parameter of flow rate is 40LPM.

During any one respiratory cycle, when the gas flow rate inhaled by the patient at the proximal end is the highest, the proximal pressure reaches the minimum value. Therefore, when the proximal pressure reaches the minimum value, it means that the current gas flow rate inhaled by the patient is the peak inspiratory flow rate. If the minimum value of the proximal pressure is within the first threshold range, it indicates that the gas flow rate, which can be provided by the proximal end of the patient under the current first gas flow rate, approaches or exceeds the inspiratory peak flow rate of the patient. Therefore, this first gas flow rate can be used as a reference for determining the second gas flow rate. For example, the second gas flow rate should not be less than the current first gas flow rate. The first threshold range can be determined based on the clinical experience of medical staff or other physiological parameters of the patient. For example, if the first threshold range is a fixed value of 0, it means that when delivering gas(es) at multiple first gas flow rates, a first gas flow rate must be found, at which first gas flow rate, the proximal end of the patient will not generate negative pressure, that is, a gas flow rate at the proximal end of the patient is > gas leakage +peak inspiratory flow rate. This equation is also easy to understand. If the gas flow rate at the proximal end of the patient is<gas leakage+peak inspiratory flow rate, the patient not only needs to draw a gas from the respiratory pipeline, but also inhales a gas from the outside, resulting in a proximal pressure less than 0. The first threshold range can also be obtained based on positive end respiratory pressure, for example, the first threshold range is between the positive end respiratory pressure of the patient to a sum of the positive end expiratory pressure and a constant.

In some embodiments, when delivering gas(es) at any first gas flow rate, a first time length, in which gas(es) is(are)s delivered to the patient interface 131 at the first gas flow rate, is recorded, and a second time length within the first time length, in which second time length the proximal pressure is greater than a preset pressure threshold, is also recorded. Take a ratio of the first time length to the second time length as the gas characteristic value of the first gas flow rate. If the ratio falls into the preset second threshold range, it indicates that a time length, in which the proximal pressure stabilizes within a certain range, is expected, and thus indicates that a time length, in which, the gas flow rate at the proximal end of the patient stabilizes within a certain range, is expected. For example, a time length, in which, the gas flow rate approaches or exceeds the inspiratory peak flow rate of the patient, is expected. Therefore, the corresponding first gas flow rate can be used as a reference for determining the second gas flow rate.

In some embodiments, when delivering gas(es) at any first gas flow rate, an integral value of the proximal pressure over time during the inspiratory phase of the patient is obtained, and the integral value is used as the gas characteristic value. The curve in FIG. 5 shows variation(s) of proximal pressure over time under a first gas flow rate, wherein the dashed line is a baseline. An area formed by the variation curve of proximal pressure and the baseline is the integral value of the proximal pressure over time. This integral value can represent work done by the patient during the inspiratory phase. The baseline can be preset by medical staff based on the condition of the patient, for example, the baseline is generated from the calculated proximal pressure when the ventilation device 100 has not started ventilation and the patient has not started respiration after being connected to the patient interface 131. At this time, the baseline represents the proximal pressure, when the patient has not yet started respiration and the ventilation device 100 has not yet started ventilation. If the integral value falls into a preset third threshold range, it indicates that the work done by the patient during the inspiratory phase is within a reasonable range. Therefore, the first gas flow rate corresponding to this integral value can be used as a reference for determining the second gas flow rate. Of course, the baseline can also be automatically preset by the ventilation device 100.

In the above embodiments, a first gas flow rate, which corresponds with a gas characteristic value that satisfies the preset condition, is used as the characteristic parameter of flow rate. In other embodiments, the characteristic parameter of flow rate may not necessarily correspond exactly to one first gas flow rate. At this time, the first gas flow rate may correspond with more than one gas characteristic values that satisfy the preset condition. Therefore, the characteristic parameter of flow rate can be calculated based on a first gas flow rate which corresponds to multiple gas characteristic values that satisfy the preset condition. For example, if gas characteristic value(s), which correspond(s) to n first gas flow rates, all satisfies(satisfy) preset condition(s), various fitting methods can be used to obtain one gas flow rate as the characteristic parameter of flow rate, based on these n first gas flow rates.

For example, in FIG. 6, the first threshold range is the dashed part, and the minimum value of the proximal pressure is used as a pressure statistical value. When the first gas flow rate is 40LPM and the first gas flow rate is 50LPM, a minimum value of the proximal pressures falls into the first threshold range. Therefore, an average value of the two first gas flow rates can be used as the characteristic parameter of flow rate, or the two first gas flow rates can be multiplied by a preset coefficient and added together to obtain the characteristic parameter of flow rate.

In some embodiments, after step S400, the method further includes:
S500, determine whether the second gas flow rate needs to be reset, and implement Step S600 when determining that the satisfy needs to be reset, or else, continues to implement Step S300.

From step S500, it can be seen that obtaining the characteristic parameter of flow rate does not necessarily mean resetting the second gas flow rate. For example, if the current second gas flow rate is 30LPM, then the gas characteristic value under 30LPM is calculated. Then, gas(es) is(are) at the first gas flow rates of 40LPM and 50LPM, respectively; and the gas characteristic values under 40LPM and 50LPM are calculated. Finally, after determination, it is found that the gas characteristic value under 30LPM satisfies the preset condition. Therefore, the current second gas flow rate does not need to be reset. In this case, the second gas flow rate is equal to one of multiple first gas flow rates.

In some embodiments, after obtaining a new characteristic parameter of flow rate, the current newly obtained characteristic parameter of flow rate is compared with the previous obtained characteristic parameter of flow rate, and the second gas flow rate is determined as needing to be reset when the characteristic parameter of flow rate changes. For example, when the ventilation device 100 regularly delivers gas(es) at multiple first gas flow rates, if the characteristic parameter of flow rate obtained in the next cycle are the same as that obtained in the previous cycle(s), it means that the reference for setting the second gas flow rate has not changed, and naturally there is no need to reset the second gas flow rate.

Step S600, set the second gas flow rate according to the characteristic parameter of flow rate.

In some embodiments, the mode of setting the second gas flow rate according to the characteristic parameter of flow rate includes: obtaining a change value of flow rate, taking a sum of the characteristic parameter of flow rate and the change value of flow rate as the second gas flow rate, wherein the change value of flow rate can be a preset constant, and can be positive or 0. For example, if a current second gas flow rate is 30LPM and the change value of flow rate is 5LPM, and the characteristic parameter of flow rate calculated after delivering gas(es) at multiple first gas flow rates is 35LPM, then the gas flow rate at which the gas(es) is(are) delivered can be changed from 30LPM to 40LPM.

In some embodiments, the mode of setting the second gas flow rate according to the characteristic parameter of flow rate includes: obtaining a proportional parameter, multiplying the characteristic parameter of flow rate by the proportional parameter, and using the product as the second gas flow rate. Wherein, the proportion parameter can be a preset constant, and can be greater than or equal to 1. For example, if the current second gas flow rate is 20LPM and the proportional parameter is 1.2, and the characteristic parameter of flow rate calculated after delivering gas(es) at multiple first gas flow rates is 25LPM, then the gas flow rate at which the gas(es) is(are) delivered can be changed from 20LPM to 30LPM.

In some embodiments, after obtaining the characteristic parameter of flow rate, step S500 is not executed, but the power to reset the second gas flow rate is delegated to the user.

In some embodiments, after obtaining the characteristic parameter of flow rate, the characteristic parameter of flow rate is displayed on the ventilation interface. The user can consider whether to reset the second gas flow rate according to the displayed characteristic parameter of flow rate. If it is necessary to reset the second gas flow rate, the characteristic parameter of flow rate can also be used as a reference to determine whether to increase or decrease the second gas flow rate. In this process, the user can also make decisions combining other physiological parameters obtained.

In some embodiments, information, which is related to the characteristic parameter of flow rate, is outputted to indicate to the user to set the second gas flow rate. For example, if the obtained characteristic parameter of flow rate is higher than the current second gas flow rate, the user is indicated to increase the second gas flow rate appropriately. If the obtained characteristic parameter of flow rate is lower than the current second gas flow rate, the user is indicated to decrease the second gas flow rate appropriately. In addition, in addition to the regulation direction, indication for a regulation amount can also be provided. For example, when indicating the user to increase the current second gas flow rate, a suggestion for how much to increase can also be given. When indicating the user to decrease the current second gas flow rate, a suggestion for how much to decrease can also be given.

In some embodiments, after obtaining the characteristic parameter of flow rate, the current characteristic parameter of flow rate is compared with the previous obtained characteristic parameter of flow rate. If the current characteristic parameter of flow rate changes when compared to the previous obtained characteristic parameter of flow rate, information related to the changed characteristic parameter of flow rate is outputted to indicate to the user to increase or decrease the second gas flow rate. For example, when the ventilation device 100 regularly delivers gas(es) at multiple first gas flow rates, if the characteristic parameter of flow rate obtained in the next cycle increase, when compared to the characteristic parameter of flow rate obtained in the previous cycle, the user is indicated to increase the second gas flow rate.

According to the ventilation device and the method for regulating a gas flow rate thereof in the above embodiments, it is possible to more accurately determine the gas flow rate that the patient needs to inhale, thereby ensuring inspiratory requirements of the patient. For example, the peak inspiratory flow rate of the patient can be calculated during the process of receiving respiratory support, and the second gas flow rate can be determined according to the peak inspiratory flow rate, ensuring the effectiveness of respiratory support.

In the above embodiments, after calculating the characteristic parameter of flow rate for the patient, the flow rate for subsequent ventilation is automatically set according to the characteristic parameter of flow rate, or the flow rate for subsequent ventilation is regulated based on the flow characteristic as a reference. In other embodiments, as the characteristic parameter of flow rate is related to a physiological state of the patient, they can also be used in other scenarios.

In some embodiments, after calculating the characteristic parameter of flow rate, the characteristic parameter of flow rate is compared with a preset parameter threshold, and an alarm is triggered when the characteristic parameter of flow rate does not match the parameter threshold. For example, when the first gas flow rate, which corresponds to a gas characteristic value which satisfies the preset condition, is used as a characteristic parameter of flow rate, the first gas flow rate is compared with a sixth threshold range. If the first gas flow rate exceeds the sixth range, an alarm is issued.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions, which are executed on a computer or other programmable data processing apparatus, can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "including", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. A ventilation device, **characterized in that**, comprising:
a gas source interface, which is capable of being connected with a gas source;
a patient interface, which is capable of being connected with a respiratory system of a patient;
a flow rate delivery apparatus, which is configured to deliver gas(es) to the patient interface;
a pressure sensor, which is configured to acquire pressure data of a first signal acquisition point;
a flow sensor, which is configured to acquire flow rate data of a second signal acquisition point; wherein both of the first signal acquisition point and the second acquisition point are positioned between the gas source interface and the patient interface; and
a processor, which is configured to control the flow rate delivery apparatus to deliver gas(es) to the patient interface at multiple first gas flow rates, and calculate a characteristic parameter of flow rate for the patient, according to the pressure data and the flow rate data under the multiple first gas flow rates;
the processor is further configured to control the flow rate delivery apparatus to deliver gas(es) to the patient interface at a second gas flow rate, so as to provide respiratory support to the patient; wherein the second gas flow rate approaches or exceeds a gas flow rate which is inhaled by the patient, when calculating the characteristic parameter of flow rate for the patient;
wherein, the characteristic parameter of flow rate is related to a physiological state of the patient, and used as a reference for determining the second gas flow rate.

2. The ventilation device according to claim 1, **characterized in that**, in order to calculate a characteristic parameter of flow rate for the patient, according to the pressure data and the flow rate data under the multiple first gas flow rates, the processor is further configured to:
calculate proximal gas characteristics of a proximal end of the patient under the multiple first gas flow rates, according to the pressure data and the flow rate data under the multiple first gas flow rates; and determine the characteristic parameter of flow rate according to the proximal gas characteristics;
wherein the proximal end of the patient is closer to the patient than the first signal acquisition point and the second signal acquisition point.

3. The ventilation device according to claim 2, **characterized in that**, in order to calculate proximal gas characteristics of a proximal end of the patient under the multiple first gas flow rates, according to the pressure data and the flow rate data under the multiple first gas flow rates; and determine the characteristic parameter of flow rate according to the proximal gas characteristics; the processor is further configured to:
calculate a proximal gas characteristic of the proximal end of the patient in a period of time under each first gas flow rate, according to the pressure data and the flow rate data in the period of time under each first gas flow rate; and
determine the characteristic parameter of flow rate according to the proximal gas characteristics in the period of time under the multiple first gas flow rates.

4. The ventilation device according to claim 3, **characterized in that**, in order to calculate a proximal gas characteristic of the proximal end of the patient in a period of time under each first gas flow rate, the processor is further configured to:
input the pressure data and the flow rate data in the period of time under each first gas flow rate into a preset model;
calculate a proximal pressure of the proximal end of the patient in the period of time under each first gas flow rate; and
use the proximal pressure as the proximal gas characteristic.

5. The ventilation device according to claim 4, **characterized in that**, in order to determine the characteristic parameter of flow rate according to the proximal gas characteristics in the period of time under the multiple first gas flow rates, the processor is further configured to:
calculate a gas characteristic value which corresponds to each first gas flow rate according to the proximal gas characteristic in the period of time under each first gas flow rate;
determine whether the gas characteristic value satisfies a preset condition, and use a first gas flow rate, which corresponds to the gas characteristic value that satisfies the preset condition, as the characteristic parameter of flow rate; or calculate the characteristic parameter of flow rate, according to a first gas flow rate which corresponds to multiple gas characteristic values that satisfy preset condition(s).

6. The ventilation device according to claim 5, **characterized in that**, in order to calculate a gas characteristic value which corresponds to each first gas flow rate according to the proximal gas characteristic in the period of time under each first gas flow rate, the processor is further configured to:
perform a statistical analysis on the proximal pressure in the period of time under each first gas flow rate, so as to obtain a pressure statistical value; and use the pressure statistical value as the gas characteristic value; wherein the pressure statistical value is any one of a mean value, a standard deviation value, a median value, a maximum value, and a minimum value;
preferably, in order to determine whether the gas characteristic value satisfies a preset condition, the processor is further configured to:
determine whether the pressure statistical value falls into a preset first threshold range.

7. The ventilation device according to claim 5, **characterized in that**, in order to calculate a gas characteristic value which corresponds to each first gas flow rate according to the proximal gas characteristic in the period of time under each first gas flow rate, the processor is further configured to:
record a first time length for the flow rate delivery apparatus to deliver gas(es) to the patient interface at each first gas flow rate;
record a second time length in which the proximal pressure exceeds a preset pressure threshold, when the flow rate delivery apparatus delivers gas(es) to the patient interface at each first gas flow rate; and
use a ratio of the second time length to the first time length as the gas characteristic value;
preferably, in order to determine whether the gas characteristic value satisfies a preset condition, the processor is further configured to:
determine whether the ratio falls into a preset second threshold range.

8. The ventilation device according to claim 5, **characterized in that**, in order to calculate a gas characteristic value which corresponds to each first gas flow rate according to the proximal gas characteristic in the period of time under each first gas flow rate, the processor is further configured to:
obtain an integral value of the proximal pressure over time during an inspiratory phase of the patient, when the flow rate delivery apparatus delivers gas(es) to the patient interface at each first gas flow rate; and
use the integral value as the gas characteristic value;
preferably, in order to determine whether the gas characteristic value satisfies a preset condition, the processor is further configured to:
determine whether the integral value falls into a preset third threshold range.

9. The ventilation device according to claim 1, **characterized in that**, the processor is further configured to, at a predetermined interval, repeatedly:
control the flow rate delivery apparatus to deliver gas(es) to the patient interface at the multiple first gas flow rates, and calculate the characteristic parameter of flow rate according to the pressure data and the flow rate data under the multiple first gas flow rates.

10. The ventilation device according to claim 9, **characterized in that**, in order to control the flow rate delivery apparatus to deliver gas(es) to the patient interface at the multiple first gas flow rates, and calculate the characteristic parameter of flow rate according to the pressure data and the flow rate data under the multiple first gas flow rates, the processor is further configured to:
compare the flow rate data with a fourth threshold range, when the flow rate delivery apparatus delivers gas(es) to the patient interface at the second gas flow rate to provide respiratory support to the patient; and control the flow rate delivery apparatus to deliver gas(es) to the patient interface at the multiple first gas flow rates, and calculate the characteristic parameter of flow rate according to the pressure data and the flow rate data under the multiple first gas flow rates, when the flow rate data is determined to exceed the fourth threshold range; or
compare the pressure data with a fifth threshold range, when the flow rate delivery apparatus delivers gas(es) to the patient interface at the second gas flow rate to provide respiratory support to the patient; and control the flow rate delivery apparatus to deliver gas(es) to the patient interface at the multiple first gas flow rates, and calculate the characteristic parameter of flow rate according to the pressure data and the flow rate data under the multiple first gas flow rates, when the pressure data is determined to exceed the fifth threshold range.

11. The ventilation device according to any one of claims 1-10, **characterized in that**, the processor is further configured to:
set the second gas flow rate according to the characteristic parameter of flow rate.

12. The ventilation device according to claim 11, **characterized in that**, in order to set the second gas flow rate according to the characteristic parameter of flow rate, the processor is further configured to:
obtain a change value of flow rate, and take a sum of the characteristic parameter of flow rate and the change value of flow rate as the second gas flow rate;
obtain a proportional parameter, and take a product of the characteristic parameter of flow rate and the proportional parameter as the second gas flow rate; or
set the second gas flow rate according to a changed characteristic parameter of flow rate, after determining that the characteristic parameter of flow rate has been changed.

13. The ventilation device according to claim 1, **characterized in that**, using the characteristic parameter of flow rate as a reference for determining the second gas flow rate, comprises at least one of:
displaying the characteristic parameter of flow rate;
outputting information, which is related to the characteristic parameter of flow rate, so as to indicate to a user to set the second gas flow rate; and
outputting information, which is related to the changed characteristic parameter of flow rate, after the characteristic parameter of flow rate is determined to have been changed, so as to indicate to a user to increase or decrease the second gas flow rate.

14. The ventilation device according to claim 1, **characterized in that**:
in a first ventilation phase, the processor is further configured to control the ventilation device to deliver gas(es) to the patient interface at the multiple first gas flow rates, and to calculate the characteristic parameter of flow rate for the patient according to the pressure data and the flow rate data under the multiple first gas flow rates;
in a second ventilation phase, the processor is further configured to control the flow rate delivery apparatus to deliver gas(es) to the patient interface at the second gas flow rate, so as to provide respiratory support to the patient.

15. A ventilation device, **characterized in that**, comprising:
a gas source interface, which is capable of being connected with a gas source;
a patient interface, which is capable of being connected with a respiratory system of a patient;
a flow rate delivery apparatus, which is configured to deliver gas(es) to the patient interface; and
a processor, which is configured to:
acquire pressure data of a first signal acquisition point, and acquire flow rate data of a second signal acquisition point; wherein both of the first signal acquisition point and the second acquisition point are positioned between the gas source interface and the patient interface; and
control the flow rate delivery apparatus to deliver gas(es) to the patient interface at multiple first gas flow rates, and calculate a characteristic parameter of flow rate of a patient, according to the pressure data and the flow rate data under the multiple first gas flow rates; wherein the characteristic parameter of flow rate is related to a physiological state of the patient.
